# EUROPEAN PATENT APPLICATION

(11) **EP 1 270 732 A1**
(43) Date of publication of application: **02.01.2003**
(21) Application number: 01115076.0
(22) Date of filing: 21.06.2001
(51) Int. Cl.: C12N 15/87, C12N 5/10, A61K 48/00

(54) **Improved transfection of eukaryontic cells with linear polynucleotides by electroporation**

(71) Applicant: Schuler, Gerold, 91080 Spardorf (DE); N.V. Antwerpes Innovatiecentrum, 2650 Edegem (BE)
(72) Inventor: Schuler, Gerold, Prof. Dr., 91080 Spardorf (DE); Berneman, Zwi N., Prof., 2018 Antwerpen (BE); Van Tendeloo, Viggo F.I., Dr., 2500 Lier (BE); Ponsaerts, Peter, 2100 Deume (BE)
(74) Representative: Helbing, Jörg, Dr. Dipl.-Chem.

(57) **Abstract**

The present invention provides an improved method for gene delivery in eukaryontic cells by electroporation, preferably in human hematopoietic cells, particular dendritic cells. The method of the invention is superior to lipofection and passive pulsing of mRNA and to electroporation of plasmid cDNA for gene delivery, including tumor antigen loading of dendritic cells.

## Description

The present invention provides an improved method for gene delivery in eukaryontic cells by electroporation, preferably in human hematopoietic cells, particular dendritic cells. The method of the invention is superior to lipofection and passive pulsing of mRNA and to electroporation of plasmid cDNA for gene delivery, including tumor antigen loading of dendritic cells.

### Background of the invention

Dendritic cells (DC) are bone-marrow-derived leukocytes that function as professional antigen-capturing and -presenting cells for the initiation of a primary immune response *in vitro* and *in vivo* (Banchereau, J., Steinman, R.M., Nature, 392:245-252 (1998)). Given their central role in cell-mediated immunity *in vivo,* they represent highly attractive targets for molecular immunotherapy of acquired diseases, such as AIDS and cancer. Recent advances in the ex *vivo* generation of DC and the ability to modulate DC functions provide a rationale to design DC-based tumor vaccines (Avigan, D., Blood Rev., 13:51-64 (1999)). The outcome of such tumor vaccines will highly depend on the efficacy of the applied antigen-loading method for optimal stimulation of cytotoxic T lymphocytes (CTL)-mediated anti-tumor immune responses (Tarte, K., Klein, B., Leukemia, 13:653-663 (1999)). Although several reports have documented viral transfer of cDNA encoding tumor-associated antigens to load DC for induction of TAA-specific cytotoxic T lymphocytes (CTL) (Dietz, A.B., Vuk, P.S., Blood, 91:392-398 (1998); Brossart, P. et al., J. Immunol., 158:3270-3276 (1997); Specht, J.M. et al., J. Exp. Med., 186:1213-1221 (1997)) nonviral gene delivery systems for DC-based vaccines would provide a more attractive approach with clinical perspectives since safety issues and immunogenicity of the vector are reduced to a minimum.

Furthermore, it is generally known that nonviral DNA transfection methods are inefficient, particularly in nondividing cells, as there is only very limited DNA trafficking to the nucleus where transcription occurs (Luo, D., Saltzman, W.M., Nat. Biotechnol., 18:33-37 (2000)). Therefore, several groups demonstrated the feasibility of mRNA transfection as a valid alternative for nonviral gene delivery, since this strategy avoids the need for entry into the nucleus as well as the complex issues of transcriptional regulation associated with DNA vectors (Lu, D. et al., Cancer Gene Ther., 1:245-252 (1994); Kariko, K. et al., Biochim. Biophys. Acta, 1369:320-334 (1998); Sawai, K. et al., Mol. Genet. Metab., 64:44-51 (1998)). The RNA approach has several advantages that render it attractive in developing DC-based tumor vaccines. First, DC can be transfected to comparable levels as compared to transduction by recombinant viruses, such as poxviruses Kim, C. J. et al., J. Immunother., 20:276-286 (1997)) or adenoviruses (Dietz, A. B., Vuk, P.S., Blood, 91:392-398 (1998)), while circumventing the drawbacks of viral vectors (Jenne, L. et al., Gene Ther., 7:1575-1583 (2000); Jonuleit, H. et al., Gene Ther., 7:249-254 (2000)). Second, DC can be charged with the full antigenic spectrum using total mRNA instead of IVT mRNA as a source of tumor antigens without prior identification of tumor-associated antigens (Zhang, W. et al., Hum. Gene Ther., 10:1151-1161 (1999)). Moreover, RNA has a short cellular half-life and lacks the potential to integrate into the host genome, thereby obviating safety concerns, e.g. insertional mutagenesis, in the context of clinical gene therapy trials (Lu, D. et al., Cancer Gene Ther., 1:245-252 (1994); Ying, H. et al., Nat. med., 5:823-827 (1999)).

However, the problem of inefficient gene transfer and low level of expression by nonviral transfection remains (Arthur, J.F. et al., Cancer Gene Ther., 4:17-25 (1997).

Previously, we reported high-level transgene expression in proliferating CD34⁺ progenitor-derived DC (34-DC) and Langerhans cells (34-LC) using electroporation-mediated gene delivery (Van Tendeloo, V.F.I. et al., Gene Ther., 5:700-707 (1998)). In contrast, nondividing monocyte-derived DC (Mo-DC), which represent a highly accessible and widely used source of *in vitro* cultured DC, were relatively refractory to cDNA transfection techniques, either by electroporation or by lipofection.

Recently, it was shown that human DC could be transfected with RNA and were capable of inducing primary antigen-specific CTL (Nair, S. K. et al., Nat. Biotechnol., 16:364-369 (1998)). However, there are very few data on efficiency of mRNA transfer in Mo-DC using passive pulsing, lipofection or electroporation, if at all. Furthermore, the feasibility of mRNA transfection in 34-DC or 34-LC has not yet been established, let alone that the method has been adapted to Mo-DC.

Electroporation methods for the integration of naked polynucleic acids into "normal" cells (such as ... cells) are generally using the following reaction conditions (Van Tendeloo V.F.I et al., Gene Ther. 5:700-707 (1998); Van Tendeloo, V.F.I. et al., Gene Ther. 7:1431-1437 (2000); Van Bockstaele, D., Berneman, Z.N., Cytometry 41:31-35 (2000); Lurquin, P.F., Mol. Biotechnol. 7:5-35 (1997); Matthews, K.E. et al., Mol. Biotechnol., vol 48, Chapter 22, Ed.Nickoloff); Spencer, S.C., Biochem. and Biotechnol. 42:75-82 (1993)):
- a cell concentration in the range of 1 to 10x10⁶ cell/ml
- a voltage in the range of 200-350 V
- a capacitance of greater than 300 µF
- a pulse length in the range of 900 to 1500 µF

However with such "conventional" electroporation methods give only very poor RNA transfection yields if applied to Mo-DC as it was e.g. shown in Strobel, I. et al., Gene Therapy 7:2028-2035 (2000)) where we reported on the electroporation of monocyte-derived dendritic cells and tested various "conventional" parameter settings: i) the cell density was tested in the range from 2x10⁶ - 4x10⁷ cells/ml while capacitance and voltage were kept constant at 300 µF and 250V, respectively, showing that an increased cell density resulted in a decreased mortality; ii) the impact of the voltage was investigated in the range from 250-350 V, while capacitance and cell density were kept constant at 300 µF and 4x10⁷cells/ml, respectively, demonstrating that an increasing voltage resulted in a higher mortality; iii) the capacitance was evaluated in the range from 300-1500 µF while cell density and voltage were kept constant at 4x10⁷ cells/ml and 250 V, respectively. Increasing capacitance yielded in an increased mortality. Although pulse times below 22 ms increased the cell viability, only a very low heterologous gene expression was detectable. In contrast pulse times above 28 ms increased transient gene expression but resulted in a high cell loss. In conclusion, the optimal electroporation conditions for immature monocyte-derived DC were found in this previously published work as follows: i.) cell density of 4x10⁷ cells/ml; ii.) voltage of 250 V; iii.) capacitance of 300 - 500 µF and iv.) pulse times between 22-28 msec. Using these optimised electroporation conditions up to 11 % of DC were GFP⁺ after 48 h, when GFP RNA was transfected. A similar transfection efficacy was obtained using GFP DNA.

Recently we also reported that effective electroporation of *in vitro* transcribed mRNA into monocyte-derived dendritic cells is possible, but did not mention how this electroporation can be achieved (Poster at the 6^{th} Symposium on dendritic cells, Port Douglas, Australia, May 26-June 1, 2000 and at the Keystone Symposia, Taos, NM, U.S.A., March 12-18, 2001).

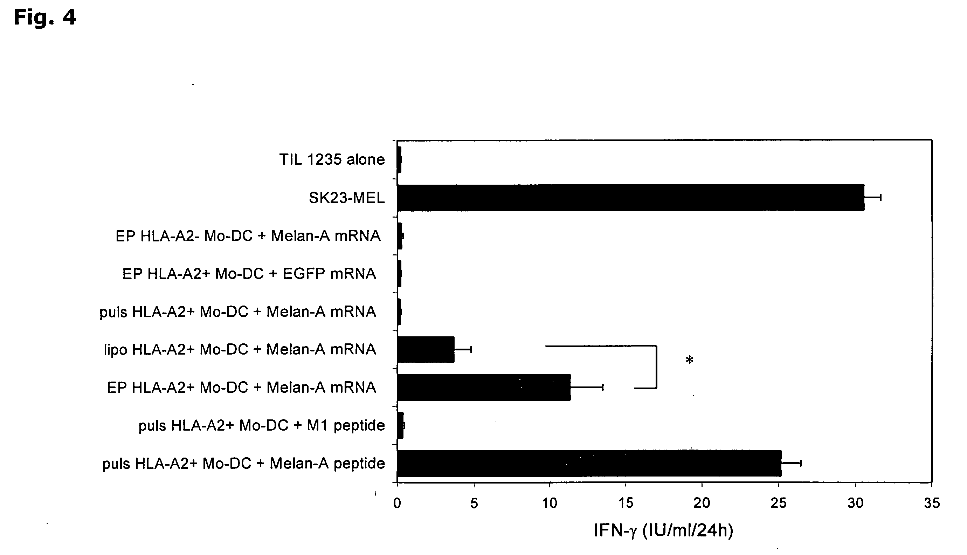

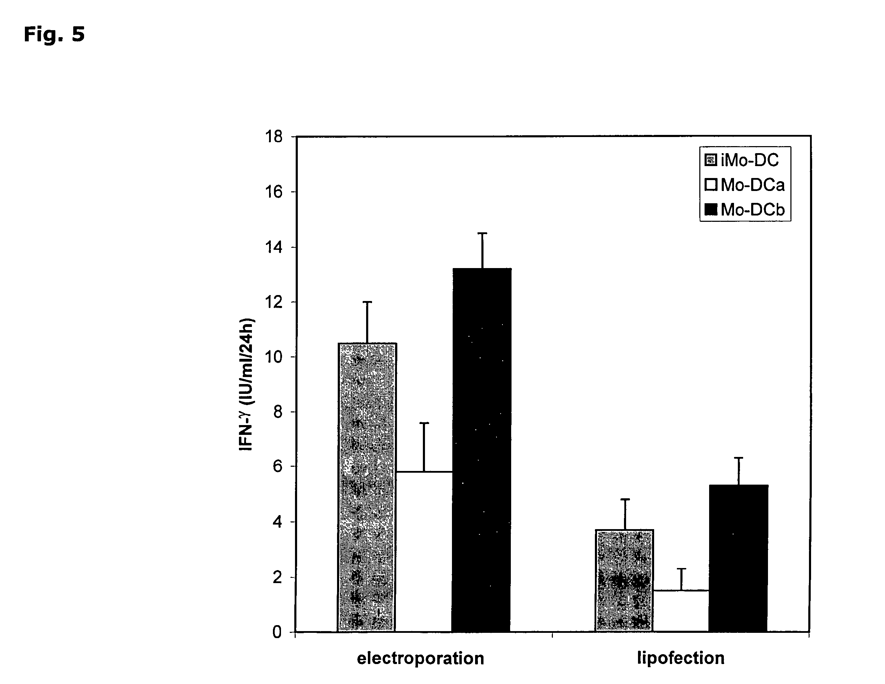

Using the novel mRNA-based electroporation, transfection efficiency in Mo-DC, 34-DC and 34-LC was at least 25, 6 and 3 times, respectively, more efficient as compared to plasmid DNA electroporation (Van Tendeloo, V.F.I. et al., Gene Ther., 5:700-707 (1998)), and also superior to previously described mRNA electroporation. Also, mRNA electroporation was superior to mRNA lipofection and passive pulsing. This increased transfection efficiency was translated in a superior biological effect *in vitro,* as confirmed by our CTL activation experiments, and could be used as a tool to investigate as to whether it results in a higher immunopotency *in vivo* (Porgador, A. et al., J. Exp. Med., 188:1075-1082 (1998)). Importantly, mRNA-transfected DC were able to efficiently process the introduced antigen and present antigenic epitopes in an MHC class I-restricted manner to a specific CD8⁺ TIL clone (Fig. 4). Furthermore, in concordance with previous reports on the effect of maturation on the antigen-presenting capacity of DC (Cella, M. et al., Curr. Opin. Immunol., 9:10-16 (1997)), antigen loading by mRNA electroporation was preferably performed prior to DC maturation in order to achieve the most optimal antigen presentation (Fig. 5), indicating the importance of the sequence of loading and DC maturation for future DC-based vaccine design Morse, M. A. et al., Cancer Res., 58:2965-2968 (1998)).

### Summary of the invention

It was now surprisingly found that with a particular electroporation setting hematopoietic cells such as monocyte derived dendritic cells can effectively be transfected with DNA and RNA. In particular, it was found out that the mRNA transfection efficiency was improved using an optimized mRNA-based electroporation. Thus, the present invention describes a method for high-efficiency non-viral transfection of Mo-DC as well as other types of dendritic cells (including CD34⁺ derived Langerhans cells and interstitial type DC by mRNA electroporation correlated with effective loading of tumor antigens into different types of human DC. The efficiency of the method of the present invention was compared with other transfection methods, such as lipofection and passive pulsing of mRNA as well as cDNA electroporation, and found to be highly superior. Furthermore, the effect of DC maturation on loading efficiency was investigated. An electroporation-based mRNA transfection protocol was developed which is suitable for highly efficient antigen loading in Mo-DC, as well as in 34-DC and 34-LC. This technique proved to be superior to mRNA lipofection or passive mRNA pulsing in terms of loading efficiency and subsequent activation of an antigen-specific CD8⁺ CTL clone.

The present invention thus provides
(1) a method for transfection of eukaryontic cells with one or more or a mixture of liner polynucleotides, which method comprises electroporation of a suspension containing the eukaryontic cells and the linear polynucleotides to be transfected at a capacitance of below 300 µF;
(2) the transfected eukaryontic cells obtainable by the method as defined in (1) above, preferably obtainable by the method as defined in (1) above;
(3) a pharmaceutical composition or vaccine comprising transfected eukaryontic cells obtainable by the method as defined in (1) above, preferably obtainable by the method as defined in (1) above;
(4) the use of the transfected eukaryontic cells obtainable by the method as defined in (1) above, preferably obtainable according to the method as defined in (1) above, for preparing an agent for immunotherapy, including induction of immunity or tolerance, or tumour therapy; and
(5) a method for immunotherapy or tumour therapy which comprises administering transfected eukaryontic cells obtainable by the method as defined in (1) above, preferably obtainable by the method as defined in (1) above, to the patient.

Designing effective strategies to load human dendritic cells (DC) with antigens such as tumor antigens) is a challenging approach for DC-based tumor vaccines. Also, the expression of other proteins (e.g., stimulatory or tolerogenic or apoptotic molecules) in DC by gene transfer might be desired, furthermore the introduction of antisense RNA by electroporation. The present invention describes a cytoplasmic expression system based on mRNA electroporation to efficiently introduce genetic information into DC. Preliminary experiments in K562 cells using an enhanced green fluorescent protein (EGFP) reporter gene revealed that mRNA electroporation as compared to plasmid DNA electroporation showed a markedly improved transfection efficiency (89% versus 40% EGFP⁺ cells, respectively) and induced a strikingly lower cell toxicity (15% death rate with mRNA versus 51% with plasmid DNA). Applying mRNA electroporation for nonviral transfection of different types of human DC, including monocyte-derived DC (Mo-DC), CD34⁺ progenitor-derived DC (34-DC) and Langerhans cells (34-LC), high-level transgene expression by mRNA electroporation was obtained in more than 50% of all DC types. mRNA-electroporated DC retained their phenotype and maturational potential. Importantly, DC electroporated with mRNA encoding Melan-A strongly activated a Melan-A-specific cytotoxic T lymphocyte (CTL) clone in an HLA-restricted manner and were superior to mRNA-lipofected or - pulsed DC. Optimal stimulation of the CTL occurred when Mo-DC underwent maturation following mRNA transfection. Strikingly, a nonspecific stimulation of CTL was observed when DC were transfected with plasmid DNA. Our data clearly demonstrate that Mo-DC electroporated with mRNA efficiently present functional antigenic peptides to cytotoxic T cells. Therefore, electroporation of mRNA encoding tumor antigens is a powerful technique to charge human dendritic cells with tumor antigens and could serve applications in future DC-based tumor vaccines. Transfection of ready mature was less efficient when maturation stimuli such as TNFα + LPS were used. The use of a certain generation methanol for Mo-DC including an optimized maturation stimulus (see Example 4) allowed, however, also for efficient transfection such as tumor antigens of mature Mo-DC.

### Description of the figures

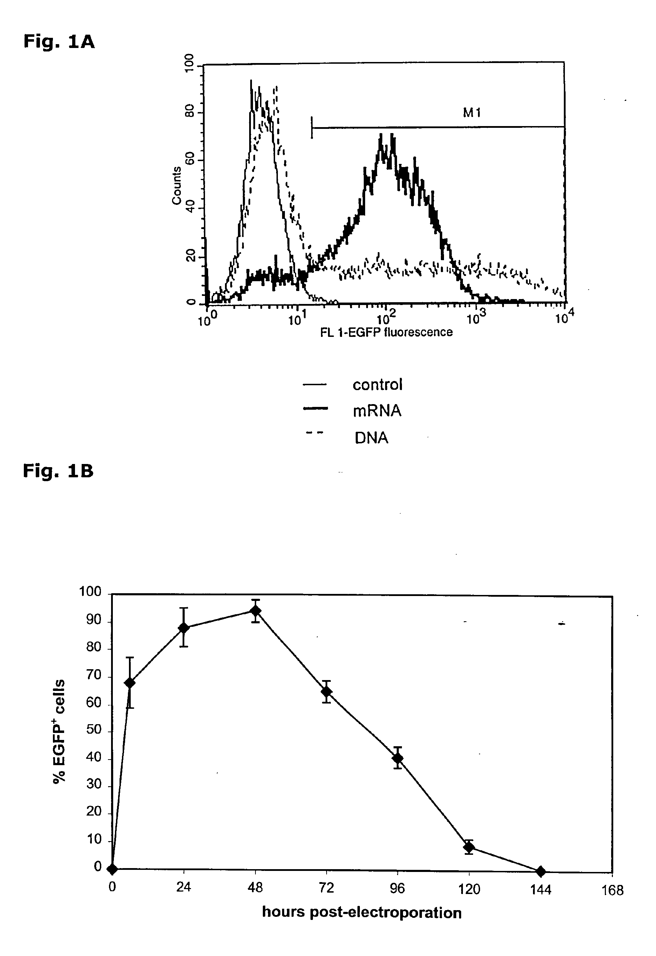

Figure 1 shows the flow cytometric analysis of transgene expression in K562 cells following EGFP mRNA electroporation.
A: K562 cells were electroporated with EGFP mRNA at 300 V, 150 µF or with EGFP plasmid DNA at 260V, 1050 µF (dashed line) as described in the Materials and Methods section. Twenty-four hours post-electroporation, flow cytometric (FCM) EGFP analysis was performed to estimate transfection efficiency of mRNA electroporation (bold line) and plasmid DNA electroporation (dashed line). An overlay histogram representative of five independent experiments is shown. Non-electroporated cells (thin line) were used to determine background fluorescence. The M1 region indicates the EGFP-positive cell fraction. The percentage of EGFP⁺ cells was 85% (bold line) and 50% (dashed line) following mRNA or plasmid DNA electroporation, respectively.
B: Kinetics of EGFP mRNA expression in K562 cells in function of time (n=3). Note the rapid induction of high-level EGFP expression already 3 hours following electroporation.

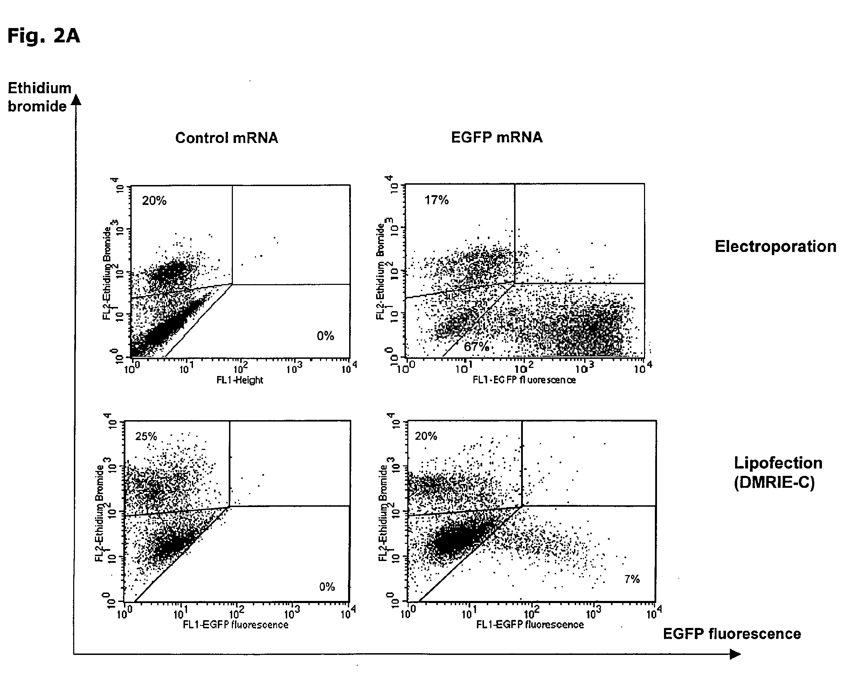

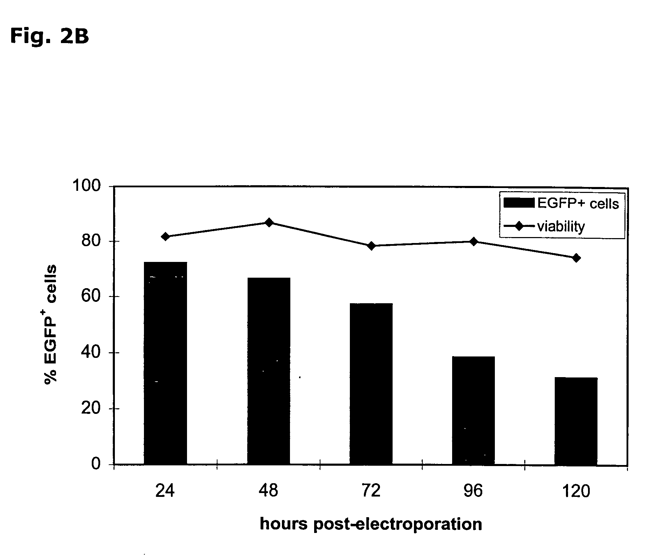

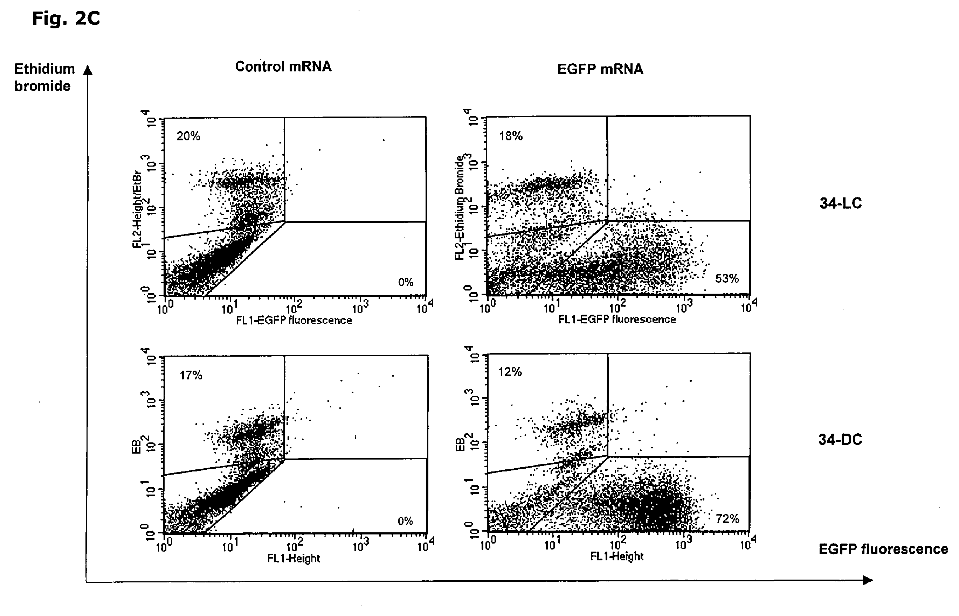

Figure 2 shows the FCM analysis of transgene expression following EGFP mRNA transfection in different types of DC.
A: Immature Mo-DC were cultured with GM-CSF and IL-4 and transfected at day 6 of culture with control (Melan-A) or EGFP mRNA by lipofection (bottom) or electroporation (top) and analyzed by FCM one day after transfection. The dot plots show EGFP fluorescence on the x-axis and ethidium bromide staining on the y-axis. Gates were drawn based on control mRNA-lipofected or electroporated Mo-DC. Percentage of dead cells (upper left corner) and viable EGFP⁺ cells (lower right corner) is indicated. Results are representative of 8 independent experiments.
B: Monitoring of EGFP mRNA expression and cell viability in Mo-DC following mRNA electroporation in function of time (n=2).
C: 34-DC (bottom) and 34-LC (top) were cultured as described in Materials and Methods and transfected at day 12 and 25 of culture, respectively, with control (Melan-A) or EGFP mRNA by mRNA electroporation. FCM analysis was performed 24 h after mRNA electroporation. The dot plots show EGFP fluorescence on the x-axis and ethidium bromide staining on the y-axis. Gates were drawn based on control mRNA-electroporated Mo-DC (left). Percentage of dead cells (upper left corner) and viable EGFP⁺ cells (lower right corner) is indicated. Results are representative of 4 independent experiments.

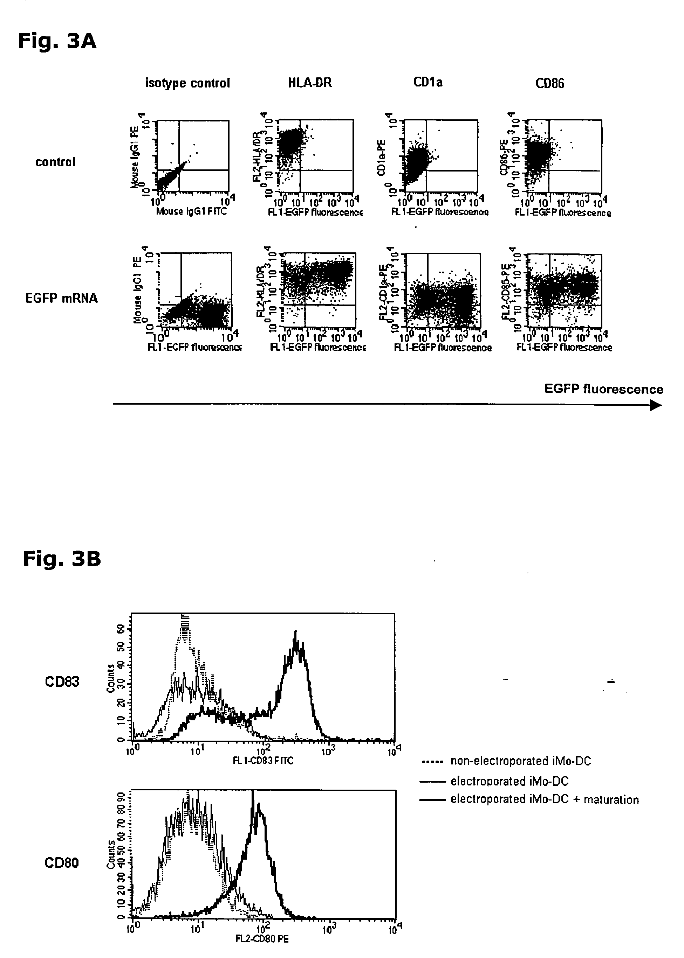

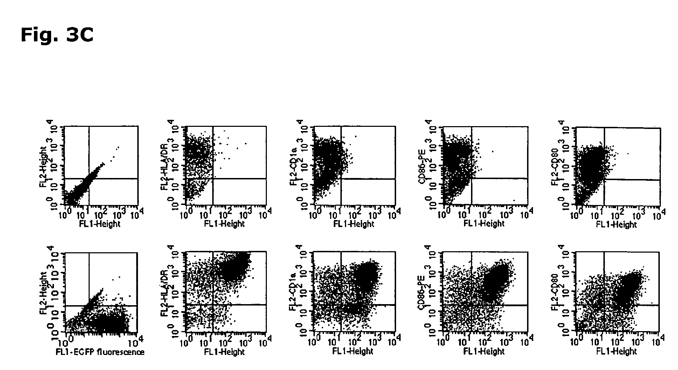

Figure 3 shows the phenotypical analysis and maturation potential of mRNA-electroporated DC.
A: Immature Mo-DC (iMo-CD) were transfected by electroporation with mRNA encoding EGFP and stained with phycoerythrin (PE)-labeled antibodies specific for CD1a, HLA-DR and CD86 one day after electroporation (bottom). Untransfected iMo-DC (top) served as controls and isotype-matched antibodies were used to set quadrants. Results are representative of 3 experiments.
B: iMo-DC were transfected by electroporation with mRNA encoding Melan-A and directly stained with a PE-labeled CD80 antibody (bottom) or indirectly stained with a CD83 antibody (top). A representative overlay histogram is shown in which the dashed line represents the control non-electroporated iMo-DC, the thin line the electroporated iMo-DC and the bold line represents electroporated iMo-DC that were allowed to mature for an additional 24 h following mRNA electroporation in the presence of TNF-α and LPS.
C: 12 day-cultured 34-DC were transfected by electroporation with mRNA encoding EGFP and stained with PE-labeled antibodies specific for CD1a, HLA-DR, CD86 and CD80 one day after electroporation (bottom). Untransfected 34-DC (top) served as controls and isotype-matched antibodies were used to set quadrants. Results are representative of 3 experiments.

Figure 4 shows the mRNA-based antigen loading of Mo-DC. Immature Mo-DC were cultured with GM-CSF and IL-4 and transfected at day 6 of culture with Melan-A mRNA by electroporation (n= 11), lipofection (n=8) or passive pulsing (n=5) or with EGFP mRNA by electroporation (n=6). The SK23-MEL melanoma cell line, HLA-A2⁺ Mo-DC pulsed with a Melan-A or irrelevant influenza peptide and HLA-A2-negative Mo-DC electroporated with Melan-A mRNA served as controls. Antigen-presenting cells (indicated on the left of the graph) were co-incubated with a Melan-A specific CD8⁺ CTL clone to determine antigen loading eficiency, as reflected by IFN-γ production of the CTL clone. Results are shown as mean ± SD. * P<0.05; EP, electroporation; lipo, lipofection; puls, passive pulsing.

Figure 5 shows the effect of DC maturation on tumor antigen presentation of mRNA-transfected Mo-DC. IFN-γ production by the CTL clone was measured after coculture with HLA-A2⁺ Mo-DC electroporated with Melan-A mRNA. iMo-DC, Mo-DC transfected at the immature stage and used as such; Mo-DCa, Mo-DC transfected at the mature stage after LPS+TNF-α stimulation; Mo-DCb, Mo-DC transfected at the immature stage, matured by LPS+TNF-α and then assayed for Melan-A-specific CTL clone stimulation. Results are shown as mean ± SD (n=4). * *P*<0.05.

Figure 6 shows the outcome of plasmid cDNA-based antigen loading of 34-LC. IFN-γ production by the CTL clone was measured after coculture with HLA-A2⁺ 34-LC electroporated with various plasmid DNA constructs encoding Melan-A (pcDNA1.1/Melan-A; n= 12), EGFP (pEGFP-N1; n=12), luciferase (pCMV-Luc; n= 3) or with a backbone vector (pcDNA1.1/Amp; n=6) lacking a eukaryotic cDNA sequence. Alternatively, 34-LC were electroporated with *in vitro* transcribed mRNA encoding EGFP or Melan-A (n=3). Results are shown as mean ± SD. * *P*<0.05.

Figure 7 shows the result of electroporation of immature monocyte-derived cells, in particular, the phenotype of dendritic cells 48 h after electroporation with GFP-RNA. The numbers in the lower right part of the quadrant indicate the EGFP-positive DC, the numbers in the upper right part show the EGFP+/CD83+ and EGFP+/CD25+ DC, respectively.

Figure 8 shows the transfection efficiency of and kinetics of EGFP expression in dendritic cells following GFP-RNA-transfection using electroporation.

Figure 9 shows the results of EGFR RNA-transfection of monocyte-derived dendritic cells by electroporation.
A: Contour plots showing the influence of voltage on cell size and granularity.
B: shows that the EGFP transfection of CD83 and CD25 is influenced by the voltage.

Figure 10: EGFP RNA-transfection of mature monocyte-derived dendritic cells by electroporation.
A and H show the transfection efficiency and kinetics of EGFP expression following GFP-RNA transfection of mature dendritic cells using electroporation.
B to G confirm that the phenotype of dendritic cells is maintained after electroporation with GFP-RNA.

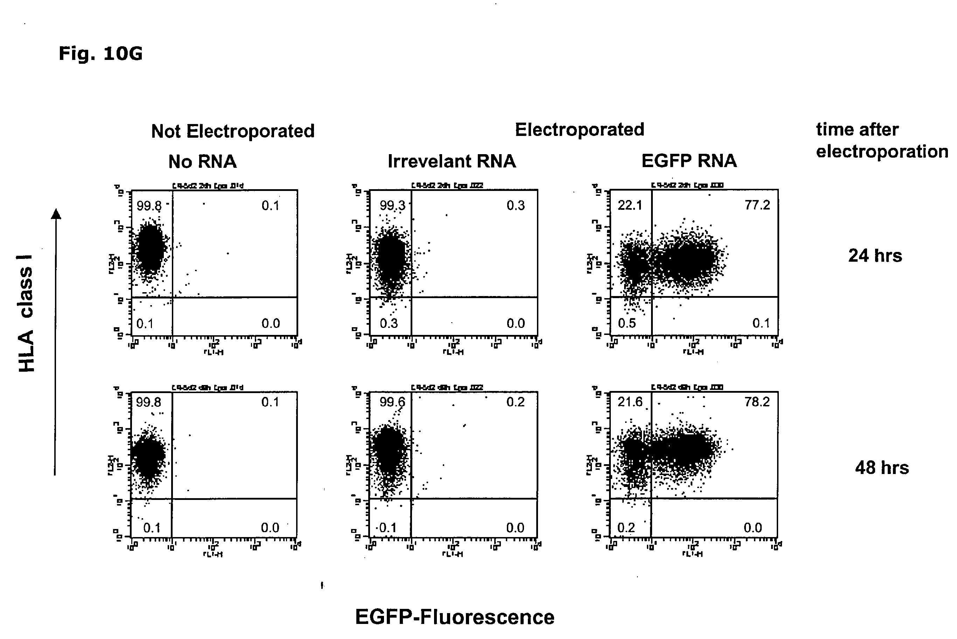

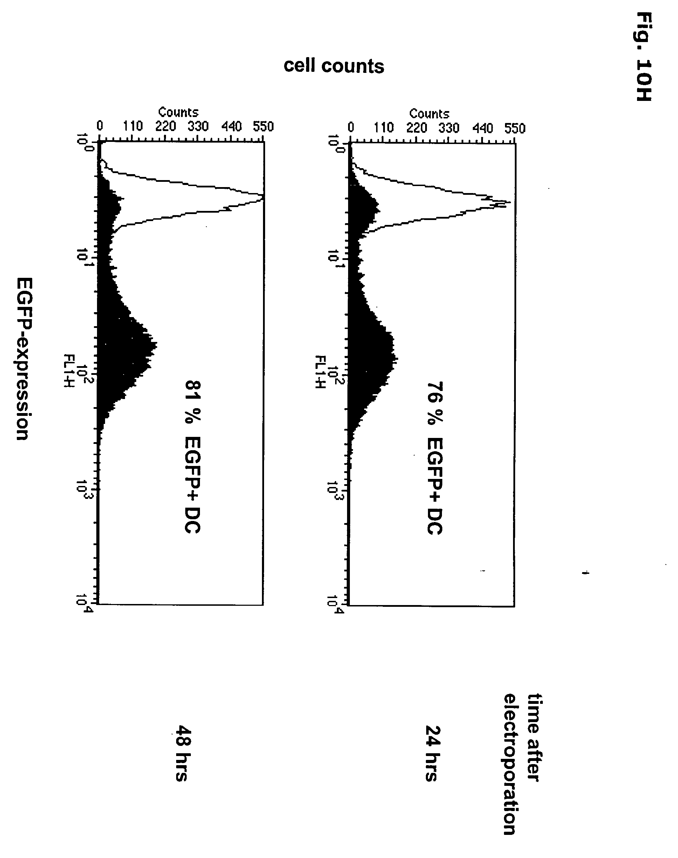

Figure 11: FCM analysis of transgene expression in immature and mature DC after EGFP mRNA electroporation in non-frozen controls and after thawing of cryopreserved samples.
The dot plots show EGFP fluorescence on the x-axis and ethidium bromide staining on the y-axis. Analysis was performed on cells exhibiting a large forward scatter and large side scatter profile, in order to allow exclusion of contaminating autologous lymphocytes. Percentage of dead cells (upper left corner), viable EGFP+ cells (lower right corner) and viable EGFP- cells (lower left corner) is indicated based on the number of dots in the quadrant analysis. (A) Dot plots show analysis of non-frozen iMo-DC 24 hours after mRNA electroporation (left), and of mRNA-electroporated iMo-DC 6 hours after thawing (middle) and 24 hours (right) after thawing. (B) Dot plots show analysis of non-frozen mMo-DC 24 hours after mRNA electroporation (left),and of mRNA-electroporated mMo-DC 6 hours after thawing (middle) and 24 hours (right) after thawing. EP, electroporation.

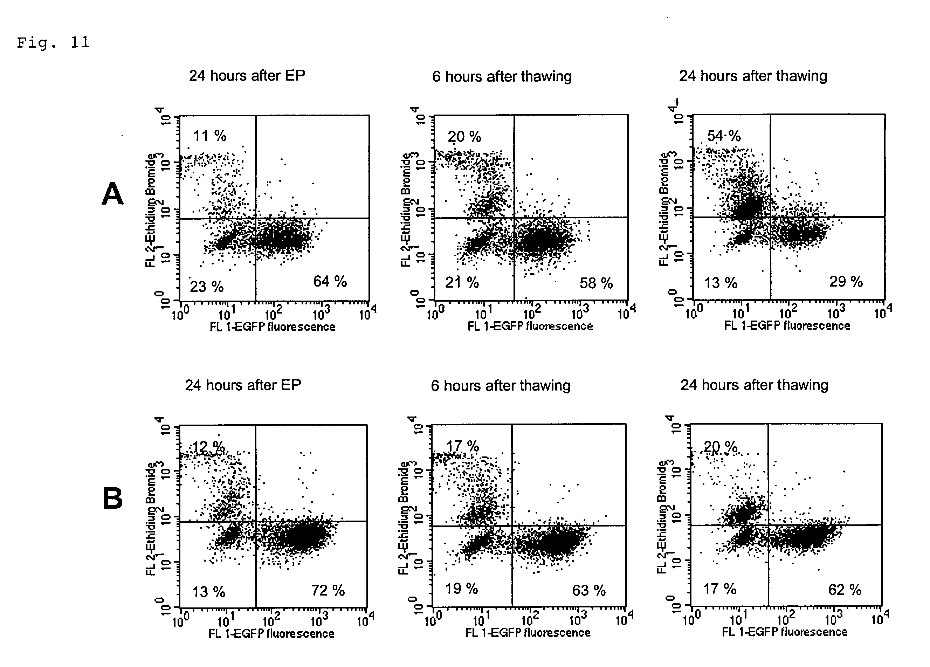

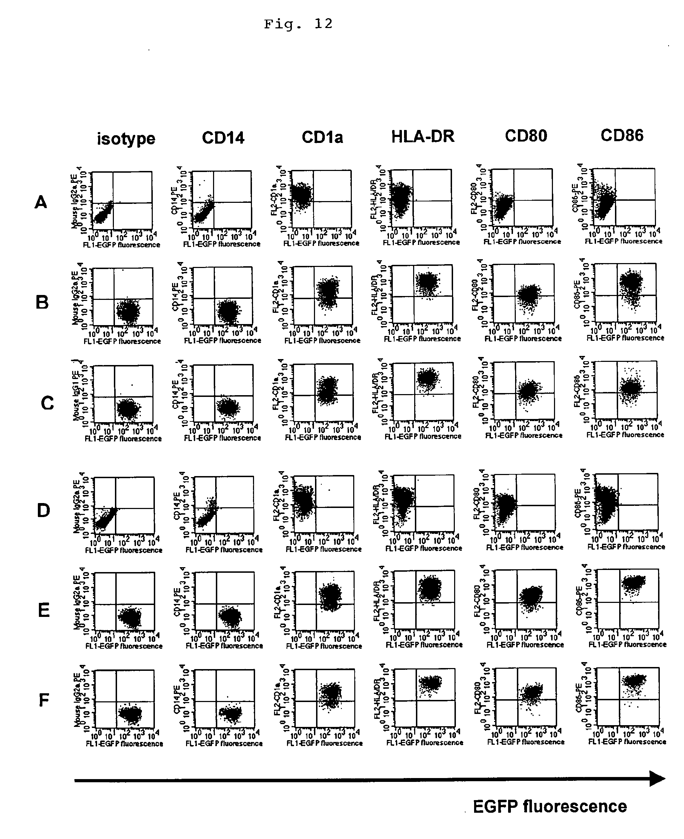

Figure 12: Representative example of phenotypical analysis of non-frozen and frozen mRNA-electroporated immature and mature DC.
Dot plots show FCM analysis of PE-labeled monoclonal antibodies directed against typical DC-markers including CD1a, HLA-DR, CD80 and CD86 (y-axis). As controls to set quadrants, isotype-matched antibodies and a PE-labeled monoclonal CD14 antibody was used. Analysis of DC markers was done on viable EGFP- cells in control samples and on viable EGFP+ cells in mRNA-electroporated DC as shown by the EGFP fluorescence on the x-axis. (A) iMo-DC on day 8 of culture, (B) EGFP+ iMo-DC after mRNA electroporation on day 6, followed by 48 hours of culture. (C) EGFP+ iMo-DC after mRNA electroporation on day 6, culture for 18 hours, cryopreservation, thawing and culture for 24 hours. (D) iMo-DC that have been stimulated for 48 hours with a maturation cocktail after day 6. (E) EGFP+ iMo-DC after mRNA electroporation on day 6 and stimulation for 48 hours with the maturation cocktail, (F) EGFP+ iMo-DC after mRNA electroporation on day 6 and culture for 24 hours with a maturation cocktail, cryopreservation, thawing and culture for 24 hours in presence of the maturation cocktail. In general, phenotyping was performed after 2 days of culture, with or without a frozen interval (that was not counted), following day 6 of the Mo-DC culture.
- As shown by the dot plot analysis (Figure 2), iMo-DC undergo maturation at 48 hours after mRNA electroporation as demonstrated by an upregulation of HLA-DR, CD80 and CD86 (Figure 2, A & B). Thawed DC have the same upregulation of HLA-DR and CD80, but have lower levels of CD86 (Figure 2, C). This is probably caused by the fact that the frozen immature DC culture is dying 24 hours after thawing. Immature Mo-DC responded well to the maturation cocktail as seen by the upregulation of HLA-DR, CD80 and CD86 in mMo-DC as compared with the expression levels in iMo-DC (Figure 2, A & D). However, the combination of mRNA electroporation and a maturation stimulus seems to be very potent in maturing DC, as this combination results in high level of HLA-DR, CD80 and CD86 expression (Figure 2, E). Frozen mature DC that were electroporated show high level maturation marker expression after thawing (Figure 2, F).

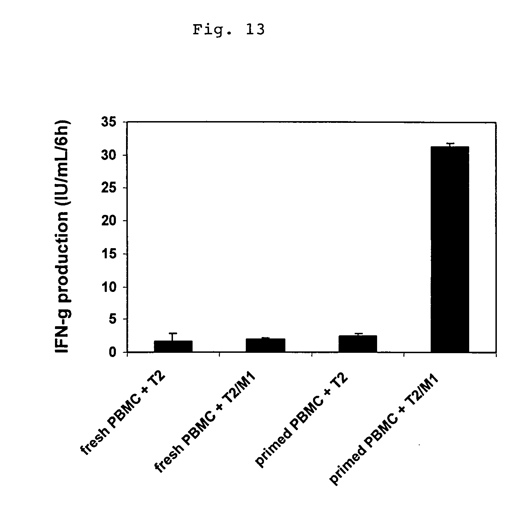

Figure 13: Stimulatory capacity of cryopreserved mRNA-electroporated mature DC.
Cryopreserved matrix protein M1 mRNA-electroporated mature DC were used as stimulators for PBMC during a 6 day coculture. Primed PBMC were then stimulated with T2 cells, pulsed with an MHC class I-restricted M1 immunodominant epitope, during a 6 hour coculture. Antigen specific T cells in the primed PBMC culture were detected as shown by positive IFN-γ production. As controls, unpulsed T2 cells were used as stimulators and fresh PBMC as responders. Results are shown as mean ± standard error.
- Upon restimulation with peptide-pulsed T2 cells, the activated T cells in the primed PBMC culture produced IFN-γ against the immunodominant matrix protein peptide. The specificity of this activation is shown by only background IFN-γ production of the primed PBMC culture against unpulsed T2 cells. To show that these cultured PBMC were stimulated during the 6 day culture, the same experiment was done with fresh PBMC. After coculture with either T2 cells or T2 cells pulsed with the peptide, no IFN-γ production was detected above background level (Figure 3).

### Detailed description of the invention

However, in the present study we demonstrate that at present the plasmid DNA electroporation approach is not applicable for tumor antigen loading of DC, because the T cell stimulation it provokes is indistinguishable from non-specific T cell stimulation mediated by plasmid DNA, either directly or indirectly. This nonspecific stimulation was not observed when using mRNA electroporation, establishing the superiority of that technique for specific DC-based T cell stimulation, at least in our hands.

For the electroporation, the following parameters were most preferred: a 4 mm cuvette with 200 µl of cell suspension and we shock the cells using 300 volts and a capacitance of 150 µF (pulse time 8-10 ms). These are optimal parameters for both leukemic K562 cells and different types of DC, both progenitor- and monocyte-derived DC. In the optimization process, other parameters were also checked, e.g., by ranging the voltage and the capacitance, as well as the volume in the cuvette, resulting in shorter or longer pulse times. In summary the following parameters for efficiency and toxicity of RNA electroporation were analyzed:
300 V - 150 µF - 200 µl - 8 ms
300 V - 300 µF - 200 µl - 10 ms
450 V - 150 µF - 200 µl - 8 ms
450 V - 300 µF - 200 µl - 10 ms
260 V - 1050 µF - 500 µl - 24 ms (typical plasmid DNA settings)
250 V - 1500 µF - 500 µl - 34 ms (typical plasmid DNA settings).

The common denominator for RNA electroporation is the low voltage (range 100 V-450 V), combined with a low capacitance (150-300 µF) (which is in contrast to DNA settings, for which a high capacitance is required) and a low electroporation volume (200 µl) to increase cell concentration.

Electroporation and incubations are all performed at room temperature and cells are resuspended in serumfree buffer (e.g. IMDM, RPMI, Opti-MEM) or in optimized electroporation buffer Opti-Mix purchased from EquiBio, UK cat n# EKIT-E1). Our electroporator type is Easyject Plus (EquiBio) which only delivers exponential decay pulses. In Examples 2-4 a Gene Pulser II (Biorad) was used.

Thus, the preferred range of voltage is 100-500 V, the range of capacitance is 150-1500 µF and the range of pulse time is 5-40 ms.

The significant decrease in toxicity observed with mRNA electroporation could in part be explained by the less stringent electrical settings required for introduction of the RNA (Table 1). Nevertheless, mRNA electroporation performed at stringent DNA settings resulted in a lower cell toxicity as well, suggesting that cell toxicity is not solely due to the electroporation procedure itself, but can also be related to the nature of the introduced nucleic acids. Moreover, co-introduction of bacterial contaminants (e.g. LPS) often found in plasmid preparations, could affect cell viability (Gordillo, G. M., Transpl. Immunol., 7:83-94 (1999)).

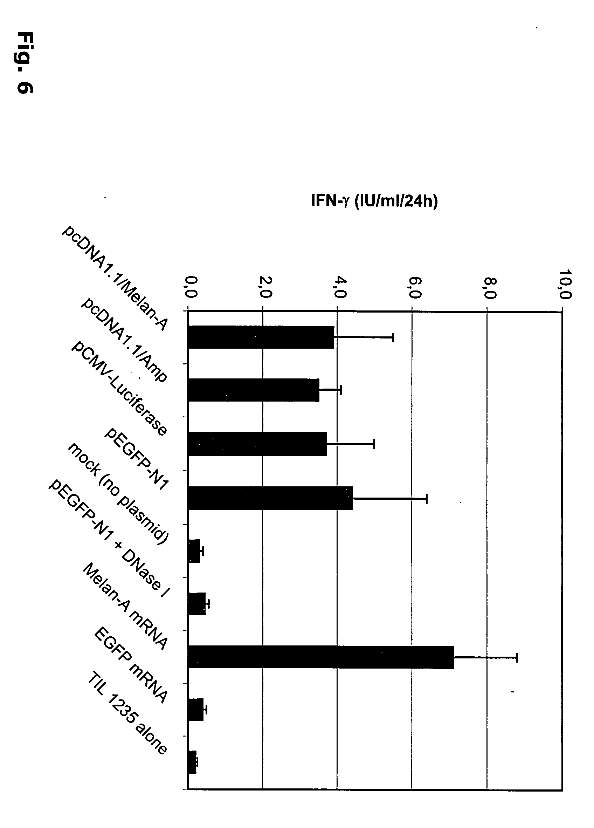

In an attempt to compare DNA and mRNA loading of DC, it was unexpectedly observed that a nonspecific stimulation of the TIL clone with plasmid DNA- but not with mRNA-electroporated 34-DC and 34-LC (Fig. 6), which could be abolished by DNase I treatment of the plasmid DNA. Although this stimulatory effect of plasmid DNA confounded data interpretation, the impact of this phenomenon on DC loading with respect to antigen-presenting capacity needs further investigation. Possible involvement of immunostimulatory sequences present in plasmid DNA (i.e. unmethylated CpG motifs) should be considered (Klinman, D. M. et al., Proc. Natl. Acad. Sci. USA, 93:2879-2883 (1996); Klinman, D. M. et al., Vaccine, 17:19-25 (1999)).

Although mRNA lipofection was overall less efficient than mRNA electroporation for loading DC, especially 34-DC and 34-LC, these data were derived from experiments with only one cationic lipid, i.e. DMRIE-C. Therefore, we cannot exclude the possibility that other lipids would accomplish comparable, or even higher, efficiencies of transfection and/or of MHC class I-restricted antigen loading of Mo-DC, 34-LC or 34-DC as compared to mRNA electroporation. Using passive mRNA pulsing, we were not able to detect any EGFP expression nor CTL activation by any type of DC examined. Therefore, these results seem somewhat in contrast to the findings of Nair et al., Nat. Biotechnol., 16:364-369 (1998) who showed in pulsing experiments that immature Mo-DC can take up mRNA without the use of a transfection agent, and subsequently prime tumor-specific CTL *in vitro.* It is possible that in the Experiment of Nair et al. passive RNA pulsing of DC lead to effective RNA transfection in a substantial portion of DC as protein expression cannot be deleted.

In conclusion, it is shown that IVT mRNA-based electroporation is a highly efficient and simple nonviral method to gene-modify human Mo-DC, 34-DC and 34-LC with tumor antigens. The technique described in this study can serve applications in DC-based tumor vaccine development and in other gene transfer protocols requiring high-level short-term transgene expression in hematopoietic cells.

### Examples

### Materials and methods

### Cell lines :

T2 cells (TAP-deficient, HLA-A2⁺, TxB hybrid), EBV-LG2 (HLA-A2⁻ EBV-transformed B lymphocytes), and SK23-MEL (Melan-A⁺ HLA-A2⁺ melanoma cell line) were kindly provided by Dr. Pierre Van der Bruggen (Ludwig institute for Cancer Research, Brussels, Belgium). K562 cells were obtained from the American Type Culture Collection (ATCC n° CCL-243, Rockville, MD, USA). Cell lines were cultured in complete medium consisting of Iscove's medium (IMDM) supplemented with L-glutamine (2 mM), penicillin (100 U/ml), streptomycin (100 µg/ml), amphotericin B (1.25 µg/ml Fungizone) and 10% fetal calf serum (FCS; Sera Lab, Sussex, UK). Cells were maintained in logarithmic phase growth at 37°C in a humidified atmosphere supplemented with 5% CO₂. All cell culture reagents were purchased from Gibco BRL (Paisley, UK).

### Melan-A-specific CTL clone:

The CD8⁺ TIL 1235 clone recognizing the immunodominant HLA-A0201-restricted Melan-A₂₇₋₃₅ epitope (AAGIGILTV) was a kind gift of Dr. J. Wunderlich (NIH, Bethesda, USA) and was cultured as described earlier with minor modifications (Reeves, M. E. et al., Cancer Res., 56:5672-5677 (1996)). Briefly, the TIL clone was maintained in AIM-V medium (Gibco BRL) supplemented with 5% pooled human AB serum (Sigma, Bornem, Belgium) and 500 IU/ml interleukin (IL)-2 (R&D Systems, Minneapolis, MN, USA) and used as responder population in DC coculture experiments.

### Source of primary cells:

Bone marrow (BM) samples were aspirated by sternal puncture from hematologically normal patients undergoing cardiac surgery, after informed consent. Peripheral blood mononuclear cells (PBMC) were obtained from healthy volunteers or hemochromatosis patients. Mononuclear cells were isolated by Ficoll-Hypaque gradient separation (LSM, ICN Biomedicals Inc., Costa Mesa, CA, USA).

### CD34⁺ cell sorting:

After Ficoll-Hypaque separation, mononuclear BM cells were indirectly stained using supernatant of the 43A1 hybridoma (anti-CD34) kindly donated by Dr. H-J. Bühring, University of Tübingen, Germany (Buhring, H. J. et al., Leukemia, 5:854-860 (1991)), followed by fluorescein isothiocyanate (FITC)-conjugated rabbit anti-mouse immunoglobulins (DAKO, Glostrup, Denmark). The CD34 labeled cells were then sorted on a FACStar^{PLUS} cell sorter (Becton Dickinson, Erembodegem, Belgium) equipped with an air-cooled argon ion laser ILT model 5500-A (Ion Laser Technology, Salt Lake City, UT, USA). Sort windows were set to include cells with low side scatter and with positive green fluorescence (CD34⁺). Purities of >95% were routinely obtained.

### In vitro culture of DC:

34-DC cultures were cultured as described previously (Lardon, F. et al., Immunology, 91:553-559 (1997)). Briefly, 1-2.10⁵ CD34⁺ cells were cultured in 2 ml of compete medium supplemented with 100 ng/ml granulocyte-macrophage colony-stimulating factor (GM-CSF; Leucomax, Novartis Pharma, Basel, Switzerland), 2.5 ng/ml tumor necrosis factor (TNF)-α (Roche Molecular Biochemicals, Mannheim, Germany) and 50 ng/ml stem cell factor (SCF; Biosource, Nivelle, Belgium) until day 5; afterwards, SCF was replaced by 1000 U/ml IL-4 (R&D Systems), which was added for the next 5-9 days. After 12 days of culture, a 15-20 fold total cell expansion was observed and cells exhibited typical markers of mature DC including CD1a, CD80, CD86 and HLA-DR (Fig. 3C).

For 34-LC, we used the protocol described by Herbst, B. et al., Blood, 88:2541-2548 (1996). Briefly, sorted CD34⁺ cells were first cultured for 8 days in complete medium containing 100 ng/ml IL-3, 100 ng/ml IL-6 and 50 ng/ml SCF (all from Biosource), followed by LC differentiation in GM-CSF (100 ng/ml) and IL-4 (1000 U/ml) for the next 4 weeks. After 25 days of culture, a 75-100-fold increase in the total number of nucleated cells was observed and cells expressed high levels of CD1a and CD40, intermediate levels of HLA-DR and low levels of CD80 and CD86 and were able to efficiently take up FITC-dextran at 37°C (data not shown).
Immature monocyte-derived DC (iMo-DC) were generated from PBMC as described by Romani, N. et al., J. Exp. Med., 180:83-93 (1996).¹⁴ Briefly, PBMC were allowed to adhere in AIM-V medium for 2 h at 37°C. The non-adherent fraction was removed, and adherent cells were further cultured for 5-7 days in IMDM supplemented with 2.5% autologous heat-inactivated plasma. GM-CSF (100 ng/ml) and IL-4 (1000 U/ml) were added to the cultures every 2-3 days starting from day 0. Maturation of iMo-DC was induced by adding 2.5 ng/ml TNF-α and 100 ng/ml lipopolysaccharide (LPS; Sigma) for 24 h starting from day 6 of the Mo-DC culture.

### HLA-A typing of DC:

HLA-A2 subtyping was determined on BM-derived mononuclear cells and PBMC by indirect staining with the supernatant of the BB7-2 hybridoma (anti-HLA-A2; ATCC), followed by FITC-conjugated rabbit anti-mouse immunoglobulins (DAKO). HLA-A2 staining was analyzed by flow cytometry using a FACScan analytical flow cytometer (Becton Dickinson, Erembodegem, Belgium).

### Synthetic peptides:

The following HLA-A*0201 restricted synthetic peptides were used: influenza virus peptide (M1; amino acids (aa) 58-66, GILGFVFTL); Melan-A peptide (MA; aa 27-35, AAGIGILTV). Peptides (>95% pure) were purchased from Sigma-Genosys (Cambridge, UK). Both peptides were dissolved in 100% DMSO to 10 mg/ml, further diluted to 1 mg/ml in serum-free IMDM and stored in aliquots at -70°C. Peptides were used at a final concentration of 20 mM.

### Peptide-pulsing of DC:

T2 cells or HLA-A2⁺ iMo-DC were washed twice with IMDM and subsequently incubated with 20 µM peptide in serum-free-medium supplemented with 2.5 µg/ml β-2 microglobulin (Sigma) for 2 h at room temperature in 15-ml conical tubes. Afterwards, cells were washed twice and used as peptide controls in cytokine release assays.

### Plasmids:

For plasmid cDNA transfection, a pEGFP-N1 plasmid (CLONTECH Laboratories, Palo Alto, CA, USA) was used encoding an enhanced green fluorescent protein (EGFP) gene under the control of a CMV promoter, and pcDNA1.1/Melan-A containing the Melan-A/MART-1 gene driven by a CMV promoter (kindly provided by Dr. Pierre Van der Bruggen). pcDNA1.1/Amp (Invitrogen, Carlsbad, CA, USA) was used as a backbone control vector.

Plasmids were propagated in *E. Coli* strain DH5α (Gibco BRL) and purified on endotoxin-free QIAGEN®-tip 500 columns (Qiagen, Chatsworth, CA, USA).

### Production of in vitro transcribed (IVT) mRNA:

For *in vitro* transcriptions, plasmids were linearized, purified using a Genieprep kit (Ambion, Austin, TX, USA) and used as DNA templates for the *in vitro* transcription reaction. pcDNA1.1/Melan-A was used as such for *in vitro* transcription under the control of a T7 promoter. EGFP cDNA (a 0.8 kb HindIII-NotI fragment) was first subcloned into pcDNA1.1/Amp and subsequently cloned as a BamHI-XbaI fragment into pSP64 (Promega, Madison, WI, USA) that allows *in vitro* transcription under the control of an SP6 promoter. Transcription was carried out in a final 20-100 µl reaction mix at 37°C for 3-4 h using the SP6 MessageMachine kit (Ambion) to generate 5' m⁷GpppG-capped IVT mRNA. Purification of IVT mRNA was performed by DNase I digestion followed by LiCl precipitation and 70% ethanol wash, according to manufacturer's instructions. For each experiment, at least three different batches of mRNA were used. mRNA quality was checked by agarose-formaldehyde gel electrophoresis. RNA concentration was assayed by spectrophotometrical analysis at OD₂₆₀. RNA was stored at -80°C in small aliquots (1 µg/µl).

### Cell transfections:

Prior to electroporation, K562 cells were washed twice with serum-free IMDM and resuspended to a final concentration of 5-10x10⁶ cells/ml in Opti-MEM (Gibco BRL). After phenotypic analysis (performed in order to confirm the presence of CD1a⁺HLA-DR⁺ DC in the cultures), 34-DC, 34-LC and Mo-DC were routinely harvested after respectively 12, 25 and 6 days of culture (unless stated otherwise), washed twice with serum-free IMDM, and resuspended to a final concentration of 10-40x10⁶ cells/ml in Opti-MEM. Subsequently, 0.5 ml of the cell suspension was mixed with 20 µg of IVT mRNA, and electroporated in a 0.4 cm cuvette using an Easyject Plus device (EquiBio, Kent, UK). In K562 cells, various voltages, capacitances and electroporation volumes were compared in order to assess their effect on mRNA transfection efficiency (see Results section). Plasmid DNA electroporation was performed as previously described (Van Tendeloo, V.F.I. et al., Gene Ther., 5:700-707 (1998)). After electroporation, fresh complete medium (including cytokines for DC) was added to the cell suspension and cells were further incubated at 37°C in a humidified atmosphere supplemented with 5% CO₂.

Lipofection of mRNA was performed using the cationic lipid DMRIE-C (Gibco BRL) according to manufacturer's instructions with minor modifications (Van Tendeloo, V.F.I. et al., Gene Ther., 5:700-707 (1998)). Briefly, K562 cells were washed twice with serum-free IMDM and resuspended to a final concentration of 1-2.10⁶ cells/ml in Opti-MEM. 34-DC, 34-LC and Mo-DC were harvested after respectively 12, 25 and 6 days of culture, washed twice with serum-free IMDM, and resuspended to a final concentration of 1-2.10⁶ cells/ml in Opti-MEM. Five µg of IVT mRNA, diluted in 250 µl Opti-MEM, was mixed with DMRIE-C, also diluted in 250 µl Opti-MEM, at a lipid:RNA ratio of 4:1. After 5-15 min of incubation at room temperature in order to allow RNA-lipid complexation, lipoplexes were added to the cells and allowed to incubate for 2 hours at 37°C. Alternatively, 5-20 µg of IVT mRNA was pulsed to the cells in the absence of DMRIE-C for 3-4 h at 37°C. Plasmid DNA lipofection was performed as described previously (Van Tendeloo, V.F.I. et al., Gene Ther., 5:700-707 (1998)). After lipofection or passive pulsing, fresh complete medium (including cytokines for DC) was added to each well.

### EGFP analysis:

EGFP-transfected cells were checked for EGFP expression 24-48 h after transfection by flow cytometric (FCM) analysis. Briefly, cells (1-5 x 10⁵) were washed once in phosphate-buffered saline (PBS) supplemented with 1% FCS and resuspended in 0.5 ml of PBS supplemented with 1% BSA and 0.1% sodium azide. Ethidium bromide (EB) at a final concentration of 10 µg/ml was added directly prior to FCM analysis on a FACScan analytical flow cytometer (Becton Dickinson) to assess cell viability. For EGFP analysis in DC cultures, gating was performed on cells exhibiting a large forward scatter (FSC) and side scatter (SSC) profile, i.e. DC, in order to allow exclusion of contaminating autologous lymphocytes. Gated DC were then evaluated for EGFP expression.

### Immunophenotyping of DC:

Immunophenotyping was performed as described previously (Van Tendeloo, V.F.I. et al., Gene Ther., 5:700-707 (1998)). The following monoclonal antibodies were used: CD1a-fluorescein isothiocyanate (FITC) (Ortho Diagnostic Systems, Beerse, Belgium), CD1a-phycoerythrin (PE) (Caltag Laboratories, San Francisco, CA, USA), CD14-PE, HLA-DR-PE, CD4-PE, CD80-PE (Becton Dickinson), CD40-FITC (BioSource, Zoersel, Belgium), CD86-PE (PharMingen, San Diego, CA, USA), CD13-FITC (DAKO) and CD83 (HB-15 clone; Immunotech, Marseille, France). Nonreactive isotype-matched antibodies (Becton Dickinson) were used as controls.

### Interferon (IFN)-γ release assay:

34-DC, 34-LC and iMo-DC were used as stimulator cells 24 h after transfection. To study the effect of maturation, 6-day-cultured iMo-DC were allowed to mature for 24 h in the presence of TNF-α and LPS prior to transfection and used as stimulators 24 h after transfection. Alternatively, iMo-DC were transfected with mRNA on day 6 of culture and, after 12-16 h to allow protein expression, TNF-α and LPS were added to induce final DC maturation. After an additional 24 h, mature transfected Mo-DC were used as stimulators. In some experiments, iMo-DC pulsed with the Melan-A or an irrelevant influenza M1 peptide were used as stimulators. Stimulators were washed twice and resuspended in AIM-V medium supplemented with 10% pooled human AB serum and 40 IU/ml IL-2. Responder CTL were washed vigorously 3-4 times and resuspended in AIM-V medium. Then, CTL (1 x 10⁵ cells) were coincubated with stimulator cells (1x10⁵ cells) in 96-round bottom plates for 24 h at 37°C in a total volume of 200 µl. Triplicate supernatant samples from these cocultures were tested for specific IFN-γ secretion by an IFN-γ ELISA (Biosource). To normalize data, the background IFN-γ secretion (defined as IFN-γ released by the CTL exposed to unmodified DC) was subtracted from each of the observed measurements. Measurements are presented as IU/ml released by 10⁵ responder cells/24 h.

### Example 1: Optimization of IVT mRNA transfection in K562 cell

In preliminary experiments to optimize mRNA electroporation, we used leukemic K562 cells, as these cells were readily transfectable by plasmid electroporation (Baum, C. et al., Biotechniques, 17:1058-1062 (1994)).¹⁵ The EGFP reporter gene was used to assess mRNA transfection efficiency. Various electroporation settings were tested and transfection efficiency was determined by flow cytometric (FCM) analysis of EGFP expression (Fig. 1A). Of all tested electrical settings, a voltage of 300 V combined with a capacitance of 150 µF in a total cuvette volume of 200 µl resulted in the highest EGFP expression (Table 1).

**Table 1.**

| Optimization of mRNA electroporation parameters in K562 cells | | | | | |
|---|---|---|---|---|---|
| **Electroporation** | | | | | |
| | voltage (V) | capacitance (µF) | cell volume (µl) | Efficiency (%) | Viability (%) |
| **DNA** | 260 | 1050 | 500 | 40 | 49 |
| | 300 | 150 | 200 | 28 | 85 |
| | | | | | |
| **RNA** | 300 | 150 | 200 | 89 | 85 |
| | 450 | 150 | 200 | 85 | 37 |
| | 300 | 300 | 200 | 83 | 59 |
| | 260 | 1050 | 500 | 81 | 73 |
| | 250 | 1500 | 500 | 80 | 69 |

| **Lipofection** | | | | | |
|---|---|---|---|---|---|
| | lipid | lipid:DNA ratio | incubation time (h) | Efficiency (%) | Viability (%) |
| **DNA** | DMRIE-C | 3:1 | 6 | 26 | 88 |
| | | | | | |
| **RNA** | DMRIE-C | 4:1 | 2 | 22 | 80 |

K562 cells were transfected as described in the Materials and Methods section by electroporation or lipofection. Cells were analyzed 24 h after electroporation by FCM for EGFP expression to estimate transfection efficiency (= % EGFP⁺ cells) as well as by ethidium bromide exclusion for cell viability. Results are the mean of four independent experiments each with a different IVT mRNA batch (standard error of the mean < 2.5%).

The vast majority of the viable cell fraction expressed EGFP to a significant extent. The percentage of EGFP-expressing K562 cells was markedly higher following mRNA electroporation than following plasmid cDNA transfection, even when cDNA electroporation was performed at optimal DNA electroporation settings, i.e. 260 V and 1050 µF (Fig. 1A). Furthermore, mRNA electroporation at optimal settings showed a significantly reduced cell mortality rate as compared to cDNA electroporation at optimal settings (15% versus 51%, respectively). DMRIE-C-mediated RNA and DNA lipofection showed a somewhat similar outcome in terms of efficiency and viability although optimal lipid:nucleic acid ratio (4:1 versus 3:1) as well as incubation time (2 h versus 6 h) varied for RNA and DNA lipofection, respectively (Table 1).
As RNA is extremely labile and has a short half-life time compared to DNA, we also studied kinetics of EGFP expression following mRNA electroporation (Fig. 1B). Transgene expression in K562 cells peaked at 24-48 h and rapidly declined to background levels after 6 days.

### Efficiency of IVT mRNA transfection in different types of DC:

Immature Mo-DC (iMo-DC) were generated from adherent PBMC in the presence of GM-CSF and IL-4. At day 5-6 of culture, Mo-DC were electroporated with EGFP mRNA. Optimization experiments revealed optimal settings similar to those of K562 cells (300 V, 150 µF), leading to maximal transfection efficiency combined with the lowest level of cell death. FCM analysis of EGFP expression showed more than 60% EGFP-expressing iMo-DC (Fig. 2A & Table 2). Mortality in Mo-DC after mRNA electroporation ranged from 15-30% (mean cell mortality rate 22±8%), although untransfected Mo-DC cultures already exhibited some degree of cell death (5-10%). When gating on the viable population, 85% of viable Mo-DC expressed EGFP to some extent. TNFα+LPS-induced maturation of Mo-DC prior to transfection showed a significant decrease in electroporation and lipofection efficiency (Table 2). DC maturation following mRNA transfection had no effect on transgene expression (data not shown). Lipofection of EGFP mRNA in Mo-DC resulted in a much lower efficiency (7.5±0.5%) and was slightly more toxic (mean cell mortality rate 28±10%) to the cells than mRNA electroporation, as shown in Fig 2A. Passive pulsing of DC with mRNA did not result in any detectable EGFP expression. Kinetic analysis of mRNA expression in Mo-DC showed a maximum 24 h after electroporation, followed by a slow decline in function of time (Fig. 2B). Five days after mRNA electroporation, EGFP was still detectable in a substantial proportion of Mo-DC (31% EGFP⁺ cells), in contrast to transgene expression kinetics in K562 cells (9% EGFP⁺ cells after 5 days). Monitoring of cell viability after mRNA electroporation revealed a somewhat stable viability in function of time (Fig. 2B).
mRNA transfection in bone marrow CD34⁺ progenitor-derived DC (34-DC) and CD34⁺ progenitor-derived Langerhans cells (34-LC) was also tested. Up to 72% and 53%, respectively, of these DC types were readily transfected by mRNA electroporation (Fig. 2C), but not by mRNA lipofection or mRNA pulsing (Table 2). Viability was always higher than 80% for both 34-DC and 34-LC (Fig. 2C). Table 2 summarizes efficiency of mRNA-based electroporation, lipofection and passive pulsing in the different types of DC.

**Table 2.**

| Efficiency of mRNA transfection in different types of DC | | | | |
|---|---|---|---|---|
| | Transfection efficiency (%) | | | |
| Method of transfection | iMo-DC | mMo-DC | 34-LC | 34-DC |
| Electroporation | 63±9 | 33±8 | 50±3 | 73±3 |
| Lipofection | 7.5±2.5 | 4±2.3 | <BG | <BG |
| Passive pulsing | <BG | <BG | <BG | <BG |

Different types of DC were transfected with IVT EGFP mRNA using electroporation, lipofection or passive pulsing. One day after transfection, EGFP expression was analyzed by FCM to estimate transfection efficiency (% EGFP⁺ DC). iMo-DC, immature Mo-DC; mMo-DC, mature Mo-DC; 34-LC, CD34⁺ progenitor-derived Langerhans cells; 34-DC, CD34⁺ progenitor-derived dendritic cells. Results are the mean ± standard deviation (SD) of at least three independent experiments for passive pulsing, lipofection or electroporation; < BG, EGFP expression below background fluorescence.

### Phenotype and maturation of mRNA-electroporated DC:

Since DC have a delicate phenotype which can easily be disturbed by culture or transfection conditions, we assessed by FCM analysis whether electroporated DC retained their respective phenotype as well as their capacity to differentiate into mature DC. Control and EGFP mRNA-transfected Mo-DC were stained using monoclonal antibodies binding to characteristic DC markers including CD1a, HLA-DR, CD80, CD86 and CD83. Electroporation of mRNA showed no effect on the phenotype of Mo-DC, as electroporated Mo-DC co-expressing EGFP retained high levels of CD1a, HLA-DR and CD86 (Fig. 3A). Mock electroporation (electroporation without mRNA) gave similar results (data not shown). The capacity of mRNA-electroporated Mo-DC to differentiate to mature Mo-DC was evaluated by expression of mature DC markers including CD80 and CD83. Fig. 3B shows that mRNA electroporation itself did not induce DC maturation, but that the maturation potential after electroporation was retained since mRNA-transfected immature Mo-DC were able to upregulate CD83 and CD80 in the presence of a maturation cocktail (TNF-α+LPS).

Also, the phenotype of 34-DC was also not affected by mRNA electroporation (Fig. 3C). EGFP⁺ 34-DC co-expressed HLA-DR, CD1a, CD80 and CD86. Similar findings were observed in 34-LC, with the exception that 34-LC exhibited lower levels of CD80 and CD86, compatible with their similarity to immature Langerhans-like DC (data not shown).

### MHC class I-restricted antigen presentation by mRNA-transfected DC:

Given the high transfection efficiency in Mo-DC, we investigated to what extent mRNA-transfected Mo-DC could process antigen and present MHC class I-restricted antigenic epitopes to an antigen-specific CTL clone. Therefore, we introduced mRNA encoding Melan-A/MART-1 into HLA-A2⁺ Mo-DC using electroporation, lipofection or passive pulsing. Mo-DC electroporated or lipofected with Melan-A mRNA markedly stimulated an HLA-A2⁺ Melan-A-specific CTL clone, as judged by IFN-γ secretion (Fig. 4). Mo-DC passively pulsed with Melan-A mRNA did not result in any CTL stimulation. HLA-A2⁺ Mo-DC electroporated with EGFP mRNA or HLA-A2⁻ Mo-DC electroporated with Melan-A mRNA did not stimulate the CTL clone to produce IFN-γ. Both HLA-A2⁺ Melan-A⁺ SK23-MEL melanoma cells and HLA-A2⁺ Mo-DC, pulsed with the immunodominant Melan-_{A27-35} peptide AAGIGILTV, were used as positive controls and induced strong IFN-γ production by the CTL clone. HLA-A2⁺ Mo-DC pulsed with the M1 influenza peptide did not elicit any specific IFN-γ production. Mo-DC electroporated with Melan-A IVT mRNA stimulated the CTL clone more than twice stronger than mRNA-lipofected Mo-DC (Fig. 4), suggesting a correlation with the difference in transfection efficiency between the two gene transfer methods (Table 2). The observation that transfection efficiency and CTL activation were correlated, was also made when comparing efficiency of mRNA electroporation (Table 2) and the capacity to stimulate the CTL clone in the other types of DC (Table 3). Electroporation of HLA-A2⁺ 34-DC and 34-LC with Melan mRNA, but not EGFP mRNA, led to specific CTL activation. In concordance with the absence of any detectable transfection level (Table 2), lipofection of 34-DC and 34-LC or passive pulsing of all types of DC with Melan-A mRNA did not result in any IFN-γ detectable above background levels (Table 3).

**Table 3.**

| CTL activation by different types of DC | | | | |
|---|---|---|---|---|
| | CTL activation (IU IFN-γ/ml/24 h) | | | |
| Method of transfection | iMo-DC | mMo-DC | 34-LC | 34-DC |
| Electroporation | 11.3±2.2 | 5.8±1.8 | 6.9±1.4 | 7.7±3 |
| Lipofection | 3.7±1.1 | 1.5±0.8 | <BG | <BG |
| Passive pulsing | <BG | <BG | <BG | <BG |

Different types of DC were transfected with IVT Melan mRNA using electroporation, lipofection or passive pulsing. One day after transfection, 10⁵ transfected DC were cocultured for 24 h with 10⁵ Melan-A-specific CTL at 37°C. Afterwards, supernatants were collected and IFN-γ secretion was checked by IFN-γ ELISA, as described in the Materials and Methods section. Results are the mean ± SD of at least five independent experiments for electroporation and of 3 independent experiments for passive pulsing and lipofection. iMo-DC, immature Mo-DC; mMo-DC, mature Mo-DC; 34-LC, CD34⁺ progenitor-derived Langerhans cells; 34-DC, CD34⁺ progenitor-derived DC; <BG, IFN-γ production below background.

### Effect of maturation on mRNA loading in Mo-DC :

Mo-DC obtained by culturing PBMC in the presence of GM-CSF and IL-4 for 5-7 days exhibit predominantly an immature phenotype (Romani, N. et al., J. Immunol. Methods, 196:137-151 (1996)). These immature Mo-DC are specialized in capturing large amounts of antigens from the environment (Sallusto, F., Lanzavecchia, A., J. Exp. Med., 179:1109-1118 (1994)). However, for optimal presentation to CTL, Mo-DC need to undergo a maturation process which can be induced by bacterial products (e.g. LPS), inflammatory cytokines (e.g. TNF-α) and/or CD40 ligation by T helper cells.¹ Therefore, in order to test whether maturation and the sequence of loading affected the antigen-presenting capacity of Mo-DC, we evaluated the ability of Mo-DC loaded with Melan-A by mRNA electroporation to stimulate the CTL clone prior to and after maturation with LPS+TNF-α. Figure 5 clearly indicates that the most potent CTL activation was obtained when mRNA loading by electroporation or lipofection was performed prior to maturation of Mo-DC. When maturation occurred prior to mRNA loading, there was a significant decrease in IFN-γ secretion by TIL cells (Fig. 5), likely to be correlated with the lower degree of transfectability of mature Mo-DC (Table 2).

### cDNA loading versus mRNA loading:

In contrast to Mo-DC, 34-LC and 34-DC can also be transfected by plasmid DNA electroporation (Van Tendeloo, V.F.I. et al., Gene Ther., 5:700-707 (1998)). Therefore, we evaluated whether plasmid DNA-transfected DC can also induce antigen-specific CTL activation. HLA-A2⁺ 34-LC electoporated with plasmid DNA or IVT mRNA encoding Melan-A were incubated with the Melan-A specific CTL to evaluate IFN-γ secretion (Fig. 6). Strikingly, we reproducibly obtained similar IFN-γ levels with Melan-A cDNA- as with control vector-transfected 34-LC, indicating nonspecific CTL stimulation. Transfection with two other irrelevant plasmids (pEGFP-N1 and pCMV-Luciferase) resulted in a similar nonspecific CTL stimulation. This phenomenon was never observed in mock-transfected (electroporation without plasmid DNA) 34-LC or when the DNA was digested by DNase I prior to electroporation (Fig. 6). Similar observations were made in 34-DC (data not shown).

### Example 2: EGFP RNA-transfection of immature monocyte-derived dendritic cells (generated from leukapheresis products and matured by a cocktail of IL-1β + IL-6 + TNFα + PEG₂ under GMP conditions for clinical application) by electroporation

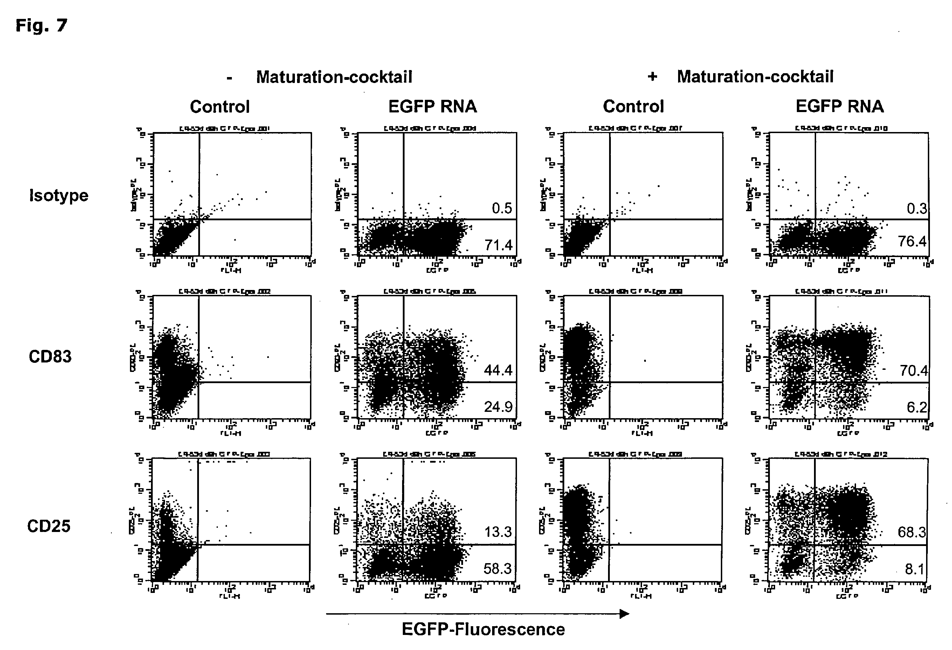

Monocyte-derived immature Dendritic Cells (DC) were generated from leukapheresis products as described (Feuerstein, B. et al., J. Immunol. Methods 245: 15-29 (2000)). Immature DC (d6) were washed twice in RPMI and once in washing-solution of the OPTIMIX®-Kit (EQUIBIO, Maidstone Kent, U.K.). DC were adjusted to a final cell concentration of 10x10⁶/ml in OPTIMIX®-Medium. Then 0,200 ml of the cell suspension were mixed with 20 µg in vitro transcribed EGFP RNA in a 1,5 ml reaction tube. After incubation at room temperature for a maximum of 3 minutes the cell suspension were transferred in a 0,4-cm-gap electroporation-cuvette. Pulse were triggered at a voltage of 300 V and a capacitance of 150 µF with the Gene Pulser II (BioRad, Munich, Germany) resulting in pulse time of 7-10 msec. Immediately after that the cell suspensions were transferred to 6-well-plates (1x10⁶ DC/ well/3 ml culture medium supplemented with GM-CSF and IL-4). In the half number of the wells terminal maturation was induced by addition of IL-1β, IL-6, TNF-α and PGE₂ as described (Feuerstein, B. et al., J. Immunol. Methods 245: 15-29 (2000)). 48 h after electroporation the DC were counterstained with the indicated mouse mAbs and PE-conjugated anti-mouse Ig followed by FACS-analysis. The results are summarised in Fig. 7.

The addition of a maturation cocktail after transfection leads to a population of Dendritic Cells that is more mature as indicated by expression of CD83 and CD25 by a much higher percentage of DC. This is important as only mature DC induce immunity in vivo while immature ones can induce tolerance (Roncarolo, M.G. et al., Exp. Med. 15;193(2):F5-9. Review. (2001)).

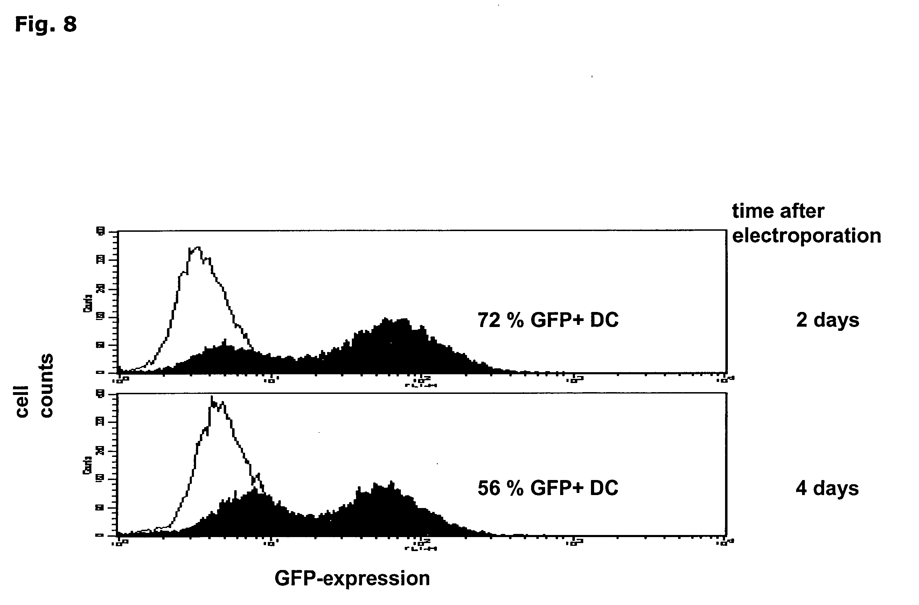

Monocyte-derived immature Dendritic Cells (DC) were processed as described above, and following addition of the maturation stimulus the longevity of EGFP expression in mature transfected DC was examined. Expression of EGFP is maintained in the majority of cells even after 4 days. The results are summarised in Fig. 8.

### Example 3: EGFP RNA-transfection of monocyte-derived dendritic cells by electroporation - Titration of Voltage

### A: Influence of voltage on cell size and granularity

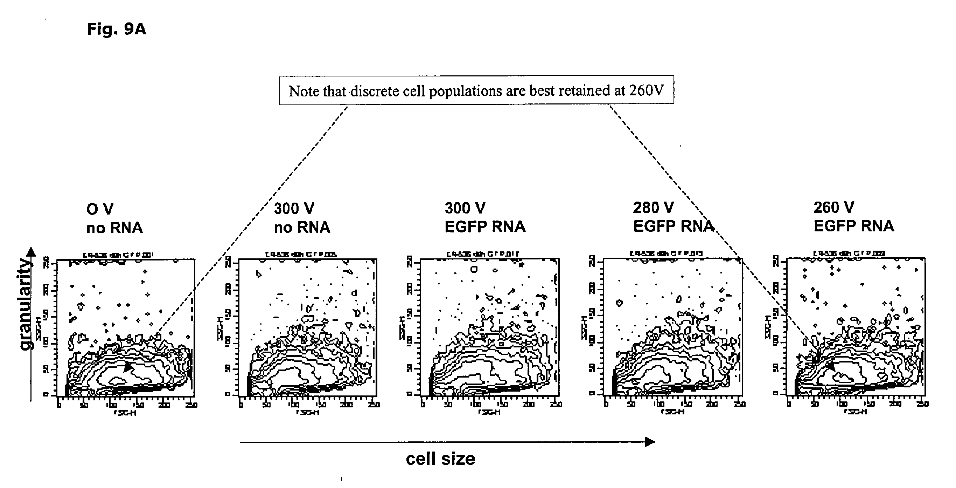

Monocyte-derived immature Dendritic Cells (DC) were generated from leukapheresis products as described (Feuerstein, B. et al., J. Immunol. Methods 245: 15-29 (2000)). Immature DC (d6) were washed twice in RPMI and once in washing-solution of the OPTIMIX®-Kit (EQUIBIO, Maidstone Kent, U.K.). DC were adjusted to a final cell concentration of 10x10⁶/ml in OPTIMIX®--Medium. Then 0,200 ml of the cell suspension were mixed with or without 20 µg in vitro transcribed EGFP RNA in a 1,5 ml reaction tube. After incubation at room temperature for a maximum of 3 minutes the cell suspension were transferred in a 0,4-cm-gap electroporation-cuvette. Pulse were triggered at the indicated voltage and a capacitance of 150 µF with the Gene Pulser II (BioRad, Munich, Germany) resulting in pulse time of 7-10 ms. Immediately after that the cell suspensions were transferred to 6-well-plates (1x10⁶ DC/ well/3 ml culture medium). Terminal maturation was induced by addition of IL-1β, IL-6, TNF-a and PGE₂ as described (Feuerstein, B. et al., J. Immunol. Methods 245: 15-29 (2000)). 48 h after electroporation the DC were analyzed. The contour-plots of Fig. 9A show on the x-axis the forward side scatter and on y-axis the sideward scatter.

The Forward and Side Scatter analysis addition reveals that for monocyte-derived Dendritic Cells that are generated from leukapheresis products, RNA-transfected by electroporation, and fully matured by adding a maturation cocktail consisting of of IL-1β, IL-6, TNF-a and PGE₂ (Feuerstein, B. et al., J. Immunol. Methods, 245: 15-29 (2000)) the use of 260 V is slightly better as the integrity of the cells is somewhat better preserved.

### B: Influence of voltage on CD83 and CD25

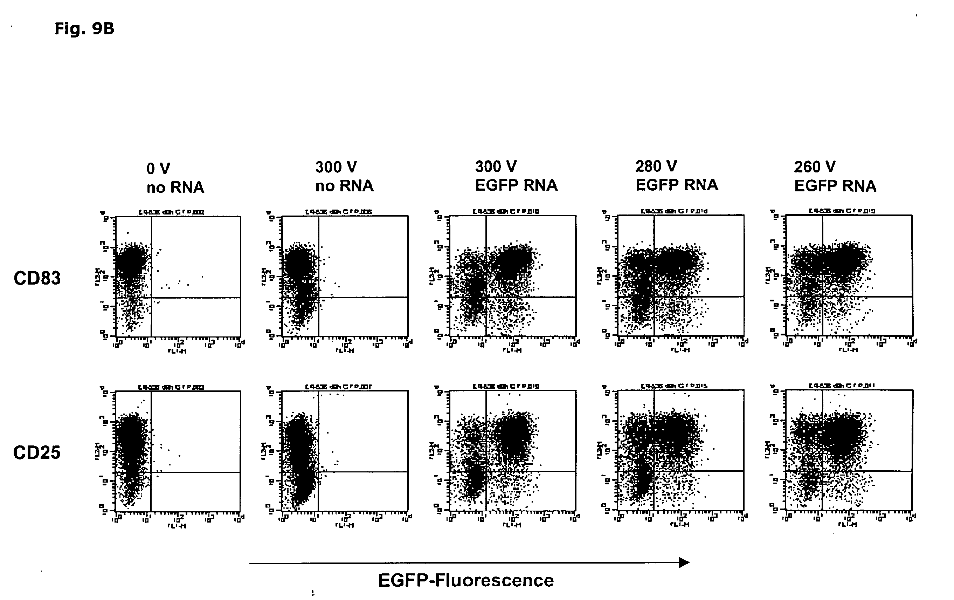

Immature DC (d6) - see Fig. 9A - were washed twice in RPMI and once in washing-solution of the OPTIMIX®-Kit (EQUIBIO, Maidstone Kent, U.K.). DC were adjusted to a final cell concentration of 10x10⁶/ml in OPTIMIX®--Medium. Then 0,200 ml of the cell suspension were mixed with or without 20 µg in vitro transcribed EGFP RNA in a 1,5 ml reaction tube. After incubation at room temperature for a maximum of 3 minutes the cell suspension was transferred in a 0,4-cm-gap electroporation-cuvette. Pulses were triggered at the indicated voltage and a capacitance of 150 µF with the Gene Pulser II (BioRad, Munich, Germany) resulting in pulse time of 7-10 msec. Immediately after that the cell suspensions were transferred to 6-well-plates (1x10⁶ DC/ well/3 ml culture medium). Terminal maturation was induced by addition of IL-1β, IL-6, TNF-a and PGE₂. 48 h after electroporation the DC were counterstained with the indicated mouse mAbs and PE-conjugated anti-mouse Ig followed by FACS-analysis. The results are shown in Fig. 9B.

The phenotypic analysis reveals that for monocyte-derived Dendritic Cells that are generated from leukapheresis products, RNA-transfected by electroporation, and fully matured by adding a maturation cocktail consisting of of IL-1β, IL-6, TNF-a and PGE₂ (Feuerstein, B. et al., J. Immunol. Methods, 245: 15-29 (2000)) the use of 260 V is slightly better as more cells are in the upper right quadrant, i.e. expressing both EGFP and the maturation markers CD83 and CD25.

### Example 4: EGFP RNA-transfection of already matured monocyte-derived dendritic cells (generated from leukapheresis cells and matured by a cocktail of IL-1β + IL-6 + TNFα + PEG₂ under GMP conditions for clinical application) by electroporation

A: Monocyte-derived immature Dendritic Cells (DC) were generated from leukapheresis products as described (Feuerstein, B. et al., J. Immunol. Methods, 245:15-29 (2000)). Immature DC (d6) were induced to undergo terminal maturation by addition of IL-1β, IL-6, TNF-α and PGE₂ as described in Feuerstein, B. et al., J. Immunol. Methods 245: 15-29 (2000). Mature DC were transfected with EGFP-RNA by electroporation as described in Example 2.

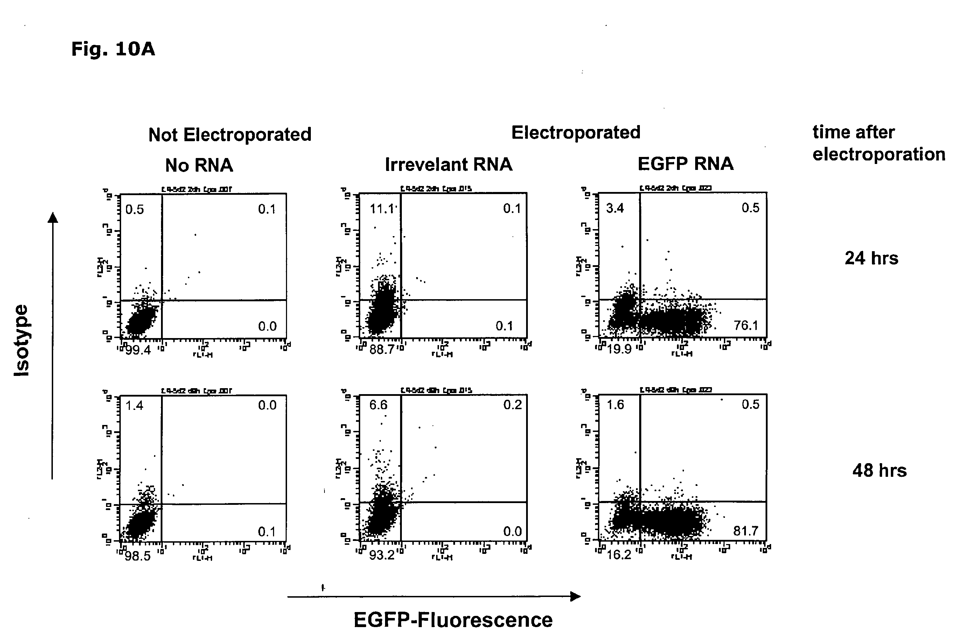

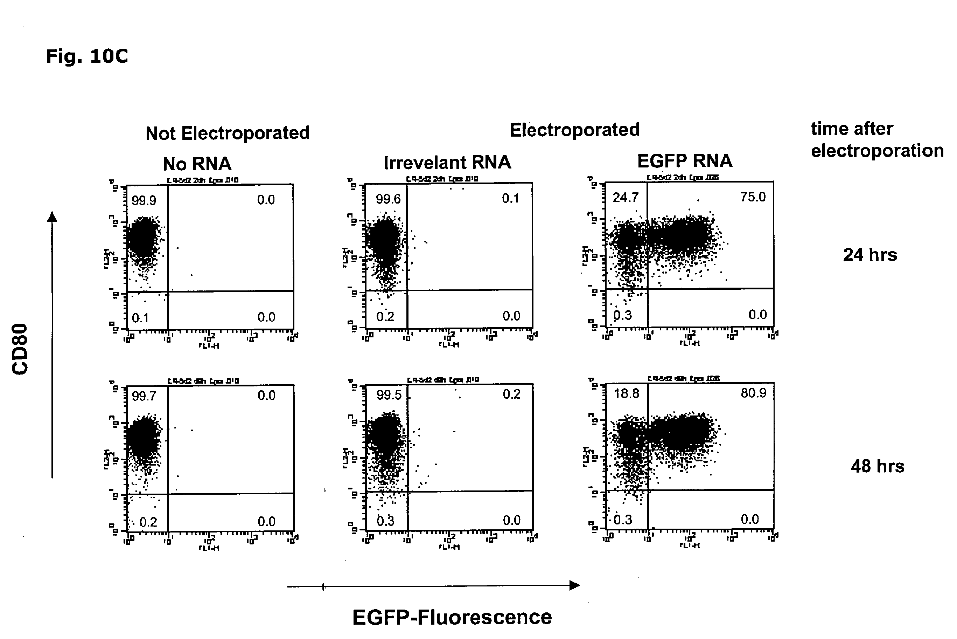

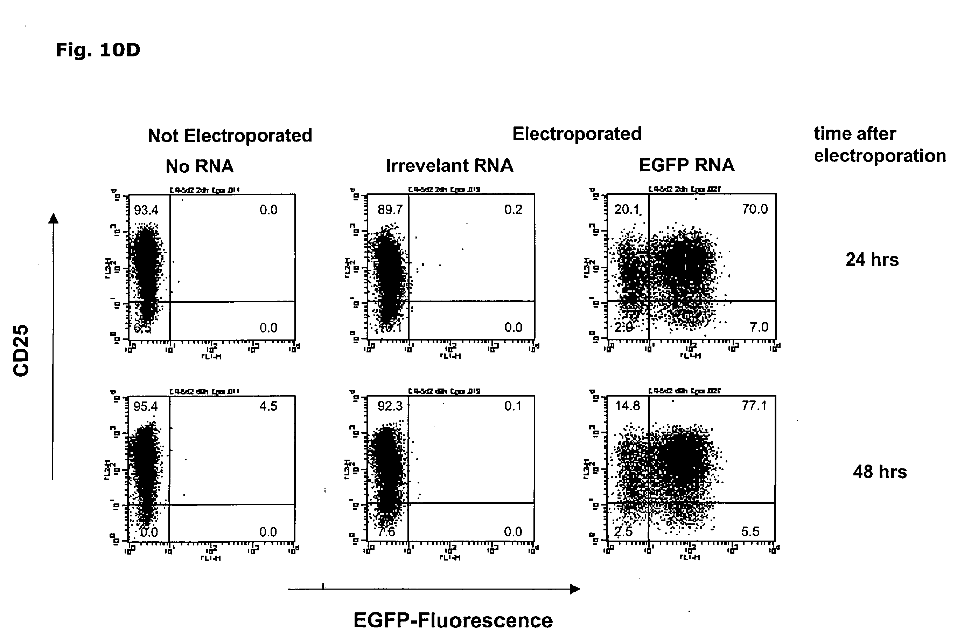

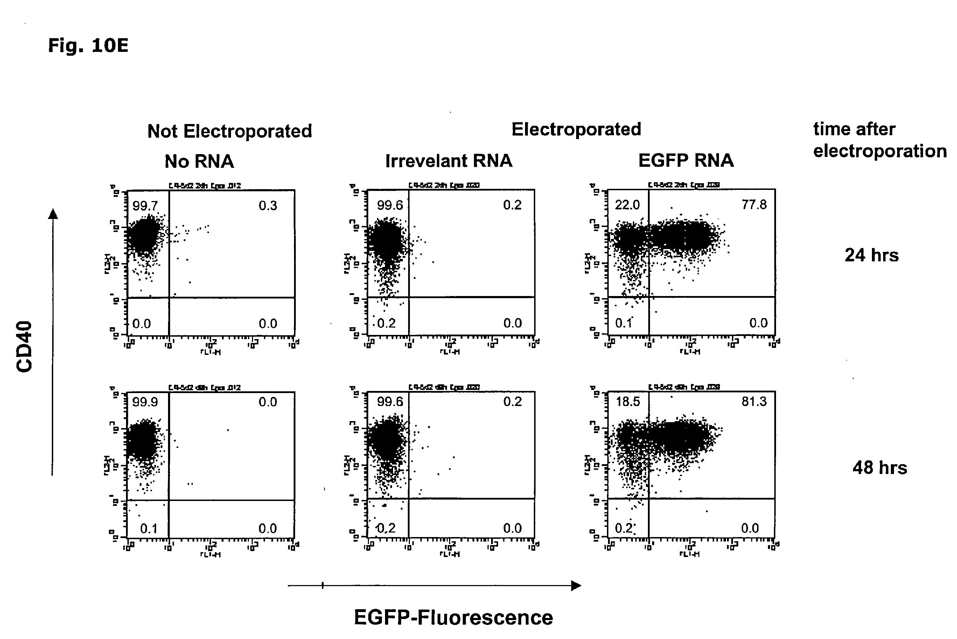

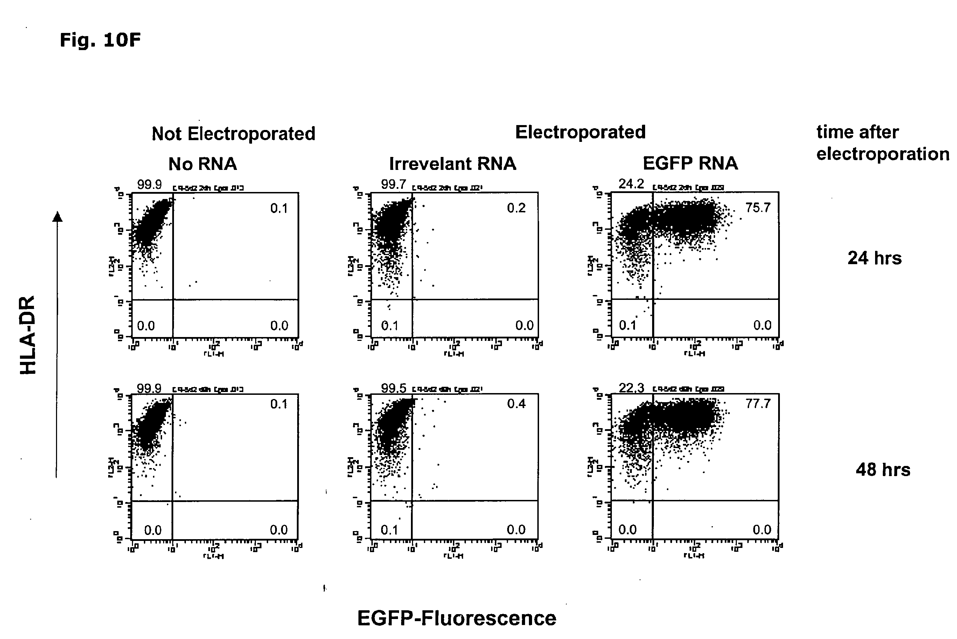

While DC matured by TNFα + LPS are transfected only to a mean of 33 %, from the results depicted in Figs. 10A and H it can be concluded that mature monocyte-derived Dendritic Cells (DC) are efficiently transfected, and maintain EGFP expression over the 48h time period tested, if ... are matured by an optimised maturation cocktail consisting of IL-1β + IL-6 + TNFα + PGE₂.

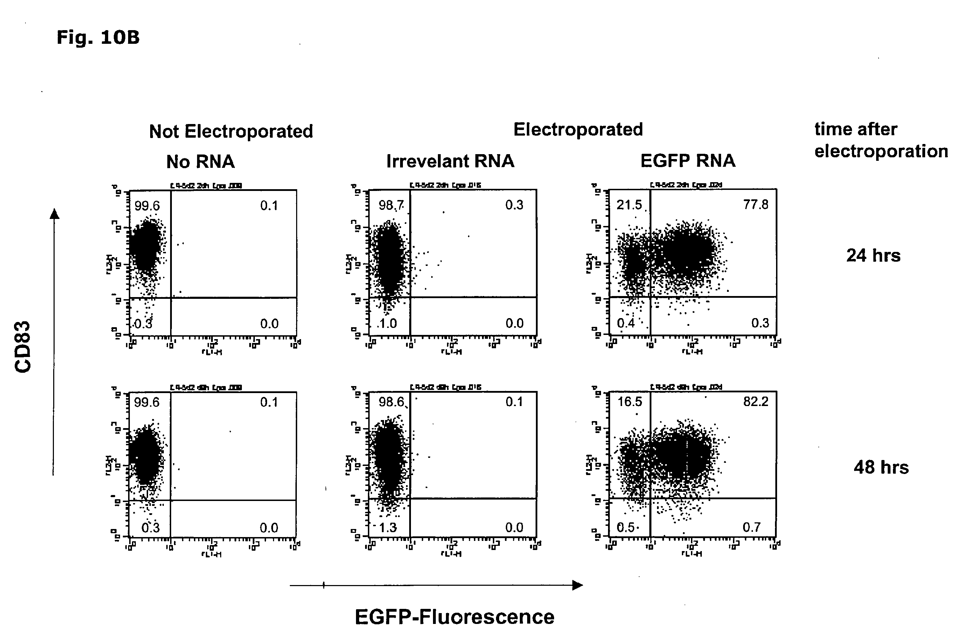

B: Mature monocyte-derived Dendritic Cells (DC) are efficiently transfected, and maintain their mature phenotype (high expression of CD83, CD80, CD25, CD40, HLA-DR and MHC class I) over the 48h time period tested as it is confirmed by Fig. 10B-G.

The addition of a maturation cocktail after transfection leads to a population of Dendritic Cells that is more mature as indicated by expression of CD83 and CD25 by a much higher percentage of DC. This is important as only mature DC induce immunity in vivo while immature ones can induce tolerance (Roncarolo, M.G et al., J. Exp. Med. 15;193(2):F5-9. Review. (2000)).

### Example 5: mRNA-electroporated mature dendritic cells retain transgene expression, phenotypical properties and stimulatory capacity after cryopreservation

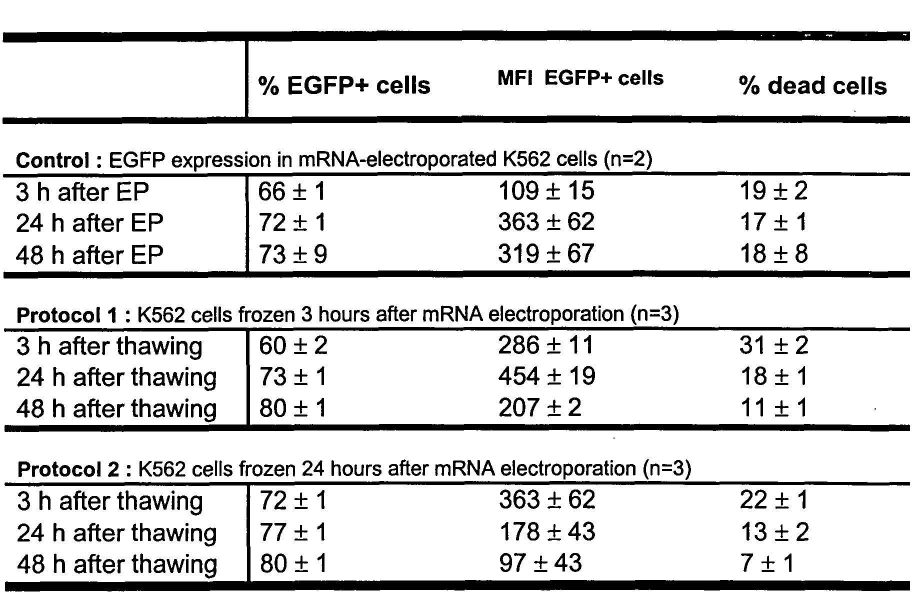

K562 cells were electroporated with EGFP mRNA and cryopreserved 3 or 24 hours after transfection. For cryopreservation, K562 cells were resuspended in cryotubes (Nunc CryoTube Vials, Nalgene Nunc International, Denmark) at a concentration of 10 x 10⁶ per mL in pure FCS. Next, the suspension was mixed on ice with an equal volume of FCS supplemented with 20% DMSO (Sigma, St. Louis, MO, USA). Cell suspensions were slowly frozen (-1°C/min) to -80°C by using a cryo freezing container (Nalgene Nunc International). Cells were frozen at -80°C for more than 24 hours before use in further experiments. Cells were analyzed at different time points before and after cryopreservation by FCM for EGFP expression to estimate transfection efficiency (= % EGFP+ cells) and the mean fluorescence intensity of EGFP⁺ cells (= MFI EGFP+ cells). The number of dead cells was determined by ethidium bromide staining (= % dead cells). Results are shown as mean ± standard error.
- There was slightly less cell survival in cultures frozen 3 hours after the electroporation as compared to cultures frozen 24 hours after electroporation (p=0.0025). Cells need to recover for a short time after the electroporation.
- Electroporated mRNA was still functional after cryopreservation. In cultures that had been frozen 3 hours after the electroporation, the MFI of expressed EGFP almost doubled between 3 and 24 hours after thawing (p=0.0009).

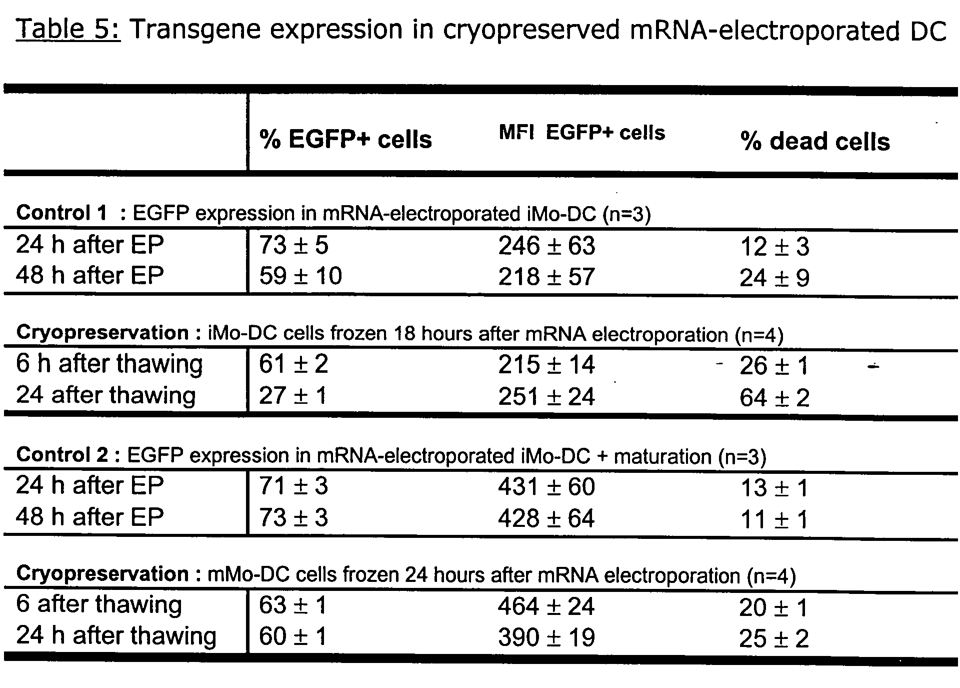

Immature Mo-DC were electroporated with EGFP mRNA. Cells were cryopreserved as immature DC 18 hours after transfection or as mature DC 24 hours after transfection. Maturation was induced by adding a maturation cocktail (TNF-α + PGE₂ + IL-1 + IL-6) directly after transfection. Cells were analyzed by FCM at different time points before and after cryopreservation for EGFP expression, in order to estimate transfection efficiency (= % EGFP+ cells) and the mean fluorescence intensity of EGFP+ cells (= MFI EGFP+ cells). The number of dead cells was determined by ethidium bromide staining (= % dead cells). Results are shown as mean ± standard error. EP, electroporation.
- As seen in a non-frozen control of immature and mature DC (Table 2, respectively control 1 and control 2), viability is not significantly affected by this electroporation in function of time (p-value respectively 0.1849 and 0.1362) and cells express high levels of EGFP (Table 2 ; Figure 1 A&B).
- Immature DC that were frozen 18 hours after electroporation seemed to survive the freezing cycle well 6 hours after thawing. There was a small increase in cell mortality (+13%, p=0.0008), but the MFI of EGFP expressing cells was approximately the same as in non-frozen control DC (p=0.5185). However, 24 hours after thawing, there was high level of cell mortality in the frozen cultures as compared to non-frozen control DC that have been cultured for 48 hours after electroporation (64% versus 24%, p=0.0017) (Table 2 ; Figure 1A).
- For the cryopreservation of mature DC, immature Mo-DC were electroporated, followed by a 2 hour incubation in medium supplemented with GM-CSF and IL-4, in order to allow transgene expression to start. Following this, the DC maturation cocktail was added and the level of EGFP expression and cell survival was determined 24 and 48 hours after transfection. DC were frozen 24 hours after mRNA electroporation and transgene expression and cell survival was determined 6 and 24 hours after thawing (Table 2 ; Figure 1 B). Six hours after thawing, DC cultures appeared to survive the freezing and have a similar number of EGFP+ cells and MFI level of EGFP+ cells as compared to non-frozen cultures (p-value respectively 0.0033 and 0.5183). Mature DC survived the thawing procedure better than frozen immature DC (64% cell death for immature DC versus 25% for mature DC after 24 hours of culture, p=0.00004).

## Claims

1. A method for transfection of eukaryontic cells with one or more or a mixture of liner polynucleotides, which method comprises electroporation of a suspension containing the eukaryontic cells and the linear polynucleotides to be transfected at a capacitance of below 300 µF.

2. The method of claim 1 , wherein
(i) the electroporation is performed at a voltage from 100 to 500 V, preferably 200 to 350 V, more preferably 250 to 300 V, and/or
(ii) the capacitance is 100 to below 300 µF, preferably 150 to 250 µF; and/or
(iii) the linear polynucleotide is a modified or unmodified, defined or undefined DNA or RNA and preferably is mRNA.

3. The method according to claim 1 or 2, wherein
- the concentration of the hematopoietic cells in the suspension is 5x10⁵ to 1x10⁹ cells per ml, preferably 1x10⁶ to 5x10⁷ cells per ml, most preferably 1 to 4x10⁷ cells per ml; and/or
- the pulsing time is from 5 to 40 ms, preferably 5 to 25 ms, and in case of transfection with acyclic polynucleotides is most preferably 7 to 10 ms; and/or
- the concentration of the polynucleotides to be transfected is ... to ... mmol/ml, preferably ... to ... ml.

4. The method according to any one of claims 1 to 3, wherein the eukaryontic cells are preferably humna cells selected from non-hematopoietic cells, which include fibroblast and tumour cells, and hematopoietic cells including, but are not limited to, mononuclear cells, marrow CD34⁺ progenitor derived dendritic cells, CD34⁺ progenitor derived Langerhans cell, monocycle-derived dendritic cells (Mo-DC), and preferably are Mo-DC including, but not limited to, immature Mo-DC arid mature Mo-DC.

5. The method of claim 1, wherein eukaryontic cells are Mo-DC, the linear polynucleotide is mRNA and the transfection is performed at a low voltage, low capacitance and a high cell concentration, preferably a voltage of 250 to 300 V, a capacitance of 150 to 250 µF and a cell concentration of 1x10⁷ to 4x10⁷ cells/ml, at a pulse time of 7 to 10 ms.

6. The method according to claim 1 or 5, wherein the acyclic and the linear polynucleotides encode tumour antigens, microbial antigens, immunostimulatory or tolerogenic molecules, anti-apoptotic molecules, adhesion and homing molecules and antigen processing molecules.

7. The method according to any one of claimes 1 to 6, wherein the eukaryontic cells are human mature Mo-DC and/or wherein the linear polynucleotide to be transfected is mRNA, and wherein the method may further comprise further maturation of the transfected MoDC by providing a maturation stimullus.

8. The method according to any one of claims 1 to 6, wherein the eukaryontic cells are human immature Mo-DC and/or wherein the linear polynucleotide to be transfected is mRNA, and wherein the method may further comprise maturation of the transfected cells by providing a maturation structure.

9. The method according to claim 7 or 8, wherein the maturation structures comprise one or more of the compounds selected from the group IL-1β, IL-6, TNF-α, PGE₂, lipopolysaccharide, immunostimulatory DNA sequences, CD40 ligand etc., and preferably is a mixture of IL-1β, IL-6, TNF-α and PGE₂.

10. The method of claims 1 to 9, wherein the method further comprises cryoconservation of the transfected cells.

11. The transfected eukaryontic cells obtainable by the method of claims 1 to 10, preferably obtainable by the method of claims 8 to 10.

12. A pharmaceutical composition or vaccine comprising transfected eukaryontic cells obtainable by the method of claims 1 to 10, preferably obtainable by the method of claims 8 to 10.

13. Use of the transfected eukaryontic cells obtainable by the method of claims 1 to 10, preferably obtainable according to the method of claims 8 to 10, for preparing an agent for immunotherapy, including induction of immunity or tolerance, or tumour therapy.

14. A method for immunotherapy or tumour therapy which comprises administering transfected eukaryontic cells obtainable by the method of claims 1 to 10, preferably obtainable by the method of claims 8 to 10, to the patient.
